(19) Europäisches Patentamt
European Patent Office
Office européen des brevets

(11) EP 2 794 798 B1

(12) EUROPÄISCHE PATENTSCHRIFT

(45) Veröffentlichungstag und Bekanntmachung des Hinweises auf die Patenterteilung:
**25.11.2015 Patentblatt 2015/48**

(21) Anmeldenummer: **12809752.4**

(22) Anmeldetag: **17.12.2012**

(51) Int Cl.:
***A61L 24/00*** (2006.01) ***C08G 18/46*** (2006.01)
***C08G 18/48*** (2006.01) ***C08G 18/73*** (2006.01)
***C08G 63/91*** (2006.01) ***C08G 18/10*** (2006.01)
***C08G 18/38*** (2006.01) ***C08G 18/42*** (2006.01)
***C09J 175/04*** (2006.01) ***A61L 24/04*** (2006.01)

(86) Internationale Anmeldenummer:
**PCT/EP2012/075825**

(87) Internationale Veröffentlichungsnummer:
**WO 2013/092506 (27.06.2013 Gazette 2013/26)**

(54) **HYDROXY-AMINOPOLYMER UND DESSEN VERWENDUNG IN POLYHARNSTOFFPOLYURETHAN-GEWEBEKLEBSTOFFEN**

HYDROXY AMINO POLYMER AND USE THEREOF IN POLYUREA/POLYURETHANE TISSUE ADHESIVES

POLYMÈRE HYDROXY-AMINO ET SON UTILISATION DANS DES ADHÉSIFS DE TISSU À BASE DE POLYURÉE POLYURÉTHANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2011 EP 11194417**

(43) Veröffentlichungstag der Anmeldung:
**29.10.2014 Patentblatt 2014/44**

(73) Patentinhaber: **Medical Adhesive Revolution GmbH**
**52074 Aachen (DE)**

(72) Erfinder:
- **HECKROTH, Heike**
**51519 Odenthal (DE)**
- **EGGERT, Christoph**
**50733 Köln (DE)**
- **HOFMANN, Jörg**
**47800 Krefeld (DE)**
- **LORENZ, Klaus**
**41539 Dormagen (DE)**
- **BROWNE, Edward**
**50672 Köln (DE)**
- **NEFZGER, Hartmut**
**50259 Pulheim (DE)**

(74) Vertreter: **Davepon, Björn**
**Patentanwaltskanzlei Davepon**
**Schlossstraße 74**
**41363 Jüchen (DE)**

(56) Entgegenhaltungen:
**EP-A1- 2 095 832 EP-A1- 2 275 466**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 794 798 B1

## Beschreibung

Die vorliegende Erfindung betrifft ein Verfahren zur Herstellung eines Hydroxy-Aminopolymers, ein Hydroxy-Aminopolymer, das nach diesem Verfahren erhältlich ist sowie ein Polyharnstoffpolyurethan-System, das ein solches Hydroxy-Aminopolymer enthält.

Gewebekleber sind in unterschiedlichen Ausgestaltungen im Handel erhältlich. Hierzu gehören die Cyanacrylate Dermabond® (Octyl-2-Cyanoacrylat) und Histoacryl Blue® (Butyl-Cyanoacrylat). Voraussetzung für eine effiziente Klebung der Cyanacrylate sind allerdings trockene Untergründe. Bei starken Blutungen versagen derartige Klebstoffe.

Als Alternative zu den Cyanacrylaten stehen biologische Klebstoffe wie zum Beispiel BioGlue®, eine Mischung aus Glutaraldehyd und Bovinem Serumalbumin, diverse kollagen- und gelatinebasierte Systeme (FloSeal®) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass sie nur bei kleineren Verletzungen auf nicht gespanntem Gewebe verwendet werden können. Kollagen- und Gelatinebasierte Systeme wie FloSeal® dienen ausschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsätzen etc. nicht möglich ist. Hier steht zur Behandlung traumatischer Wunden QuikClot® oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Aus der WO 2009/106245 A2 ist die Herstellung und Verwendung von Polyharnstoffpolyurethan-Systemen als Gewebekleber bekannt. Die hier offenbarten Systeme umfassen wenigstens zwei Komponenten. Dabei handelt es sich um einen aminofunktionellen Asparaginsäureester und ein isocyanatfunktionelles Präpolymer, das durch Umsetzung von aliphatischen Polyisocyanaten mit Polyesterpolyolen erhältlich ist. Die beschriebenen 2-Komponenten Polyharnstoffpolyurethan-Systeme können als Gewebekleber für den Verschluss von Wunden in menschlichen und tierischen Zellverbänden eingesetzt werden. Dabei kann ein sehr gutes Klebeergebnis erzielt werden.

Um eine gute Mischbarkeit der beiden Komponenten des Polyharnstoffpolyurethan-Systems sicher zu stellen, sollte die Viskosität der Komponenten bei 23 °C möglichst kleiner als 10.000 mPas sein. Eine entsprechend niedrige Viskosität weisen Präpolymere mit NCO-Funktionalitäten von weniger als 3 auf. Wenn derartige Präpolymere eingesetzt werden, ist es notwendig, als zweite Komponente einen Asparaginsäureester mit einer Aminofunktionalität von mehr als 2 einzusetzen, da ansonsten kein polymeres Netzwerk hergestellt werden kann. Dies ist jedoch erforderlich, damit das Polyharnstoffpolyurethan-System bzw. eine daraus bestehende Klebnaht die gewünschten mechanischen Eigenschaften wie Elastizität und Festigkeit aufweist. Darüber hinaus ist bei der Verwendung difunktioneller Asparaginsäureester nachteilig, dass die Aushärtungszeit bis zu 24 h beträgt, wobei das Polyharnstoffpolyurethan-System selbst nach dieser Zeit in vielen Fällen klebrig bleibt, also nicht "tack-free" ist. Ferner sind die hierbei entstehenden Klebstoffe in erster Linie für topische Anwendungen konzipiert und nicht innerhalb kurzer Zeit, beispielsweise innerhalb von 6 Monaten oder weniger im Körper biologisch abbaubar. Für eine Anwendung innerhalb des Körpers sollte ein Klebstoffsystem jedoch diese Voraussetzung erfüllen.

Aus der WO 2010/066356 sind Klebstoffsysteme für medizinische Anwendungen bekannt, in denen isocyanatterminierte Präpolymere mit sekundären Diaminen umgesetzt beziehungsweise ausgehärtet werden. Auch hier stellen sich die bereits in Bezug auf die WO 2009/106245 A2 genannten Nachteile ein.

Ferner sind Polymere bekannt, welche sowohl aminfunktionell sind als auch Hydroxylgruppen tragen (sogenannte Hydroxy-Aminopolymere). Solche Verbindungen stoßen in bestimmten Anwendungsgebieten, speziell im Bereich der Polyurethanindustrie, auf zunehmendes Interesse. Der Grund hierfür liegt darin, dass sich durch die Gegenwart zweier unterschiedlicher Typen funktioneller Gruppen, nämlich der Aminfunktionalitäten sowie der Hydroxylgruppen, neuartige Eigenschafts- und Verarbeitungsprofile erzielen lassen. So besteht beispielsweise durch die Kombination der gegenüber Isocyanatgruppen deutlich reaktiveren Aminogruppen mit den weniger reaktiven Hydroxylgruppen die Möglichkeit, den zeitlichen Verlauf von Aushärteprozessen in gewünschter Weise zu beeinflussen, was bislang bei Gegenwart nur eines Typs der vorgenannten isocyanatreaktiven funktionellen Gruppen nicht oder nur in beschränktem Maße möglich ist. Im Allgemeinen kann die Aminofunktionalität von Hydroxy-Aminopolymeren über die Addition von primären Aminen oder Ammoniak an elektronenarme Doppelbindungen, beispielsweise vom (Meth-)Acrylattyp, in Makromoleküle eingeführt werden. Die Addition von Aminen an (meth-)acrylatgruppenhaltige Polymere, u. A. an (meth-)acrylat-gruppenhaltige Polyether, ist an sich bekannt, beispielsweise werden solche Verfahren in US 5,739,192 A1, US 5,597,390 A1, US 2005/0171002 A1, DE 196 16 984 A1, DE 195 08 308 A1, WO 2010/090345 A1, JP 2009/22753 A1 und JP 04089860 A1 erwähnt.

Demgegenüber ist der Erhalt der die elektronenarmen Doppelbindungen enthaltenden Vorläuferverbindungen im Stand der Technik entweder nicht beschrieben oder erfolgt über nach statistischen Gesetzmäßigkeiten ablaufenden

Kondensationsreaktionen, beispielsweise über die Veresterung von Acrylsäure mit difunktionellen Polyethern oder die Umsetzung von Acryloylchlorid mit difunktionellen Polyethern.

Diesen beschriebenen Verfahren ist gemein, dass die Einführung der Doppelbindungen in die Vorläuferverbindungen der Hydroxy-Aminopolymere auf Kosten der Zahl der Hydroxyfunktionen erfolgt. Somit erlauben diese Verfahren es nicht, die ursprüngliche Hydroxyfunktionalität, die bei Polyethermolekülen im allgemeinen durch die Funktionalität der zur Herstellung der Polyether verwendeten Startermoleküle gegeben ist, während der Einführung der Aminofunktionen zu erhalten. Zudem ist aus den vorgenannten Druckschriften nicht bekannt, dass diese Verbindungen zum Verkleben in medizinischen Applikationen verwendet werden können.

Vor diesem Hintergrund bestand die Aufgabe der Erfindung darin, eine Isocyanat-reaktive Komponente für ein Polyharnstoffpolyurethan-System bereit zu stellen, die gut mit einem Präpolymer mit einer NCO-Funktionalität von weniger als 3 mischbar ist und mit dem Präpolymer schnell unter Ausbildung eines dreidimensionalen Polyharnstoffpolyurethan-Netzwerks umgesetzt werden kann. Ferner soll die Isocyanat-reaktive Komponente die Bereitstellung eines Polyharnstoffpolyurethan-Systems für Gewebekleber ermöglichen, die sich innerhalb kurzer Zeit nach erfolgter Wundheilung, beispielsweise innerhalb von 6 Monaten oder weniger im Körper biologisch abbauen. Dabei soll das ausgehärtete System nach ISO 10993 bei der Anwendung im Menschen keine Zytotoxizität aufweisen.

Diese Aufgabe wird gelöst durch ein Verfahren zur Herstellung eines Hydroxy-Aminopolymers umfassend die Schritte:

a) Umsetzen einer wenigstens ein Zerewitinoff-aktives H-Atom tragenden H-funktionellen Starterverbindung mit einem ungesättigten cyclischen Carbonsäureanhydrid und wenigstens einer Alkylenoxidverbindung zum Erhalt eines Hydroxylgruppen tragenden Präpolymers,

b) Addition eines primären Amins und/oder von Ammoniak an die Doppelbindung(en) des nach Schritt a) erhaltenen Hydroxylgruppen tragenden Präpolymers zum Erhalt des Hydroxy-Aminopolymers,

wobei das Verhältnis der addierten Aminogruppen zu den Hydroxylgruppen im Hydroxy-Aminopolymer wenigstens 0,6 beträgt.

Die Erfindung betrifft ferner ein Hydroxy-Aminopolymer, das nach dem erfindungsgemäßen Verfahren herstellbar ist. Aufgrund der dem erfindungsgemäßen Verfahren zugrundeliegenden polymeranalogen Umsetzung(en) stellt das Hydroxy-Aminopolymer in der Praxis in der Regel eine Mischung verschiedener Strukturen dar, die statistisch betrachtet das erfindungsgemäße Verhältnis von Aminogruppen zu Hydroxylgruppen aufweist.

Dabei ist unter dem unbestimmten Artikel "ein", "einer" usw. zu verstehen, dass wahlweise jeweils auch mehrere dieser Komponenten in dem erfindungsgemäßen Verfahren miteinander umgesetzt werden können.

Das erfindungsgemäße Hydroxy-Aminopolymer hat eine Aminofunktionalität von > 1, bevorzugt wenigstens 2, wenigstens 3 oder mehr, und ist folglich in der Lage, mit Präpolymeren einer NCO-Funktionalität von 2 oder mehr, bevorzugt von 3, schnell ein dreidimensionales Polyharnstoffpolyurethan-Netzwerk zu bilden.

Dabei hat die vorgenannte Verbindung den weiteren Vorteil, dass sie neben den Aminofunktionalitäten auch statistisch gesehen mehr als eine endständige Hydroxylgruppe trägt. Diese Gruppen sind ebenfalls NCO-reaktiv und unterstützen den schnellen Aufbau des Polymernetzwerks zusätzlich. Dieses Netzwerk zeichnet sich durch hohe Elastizität, Festigkeit, Klebstärke und einen Fehlen von Zytotoxizität aus. Außerdem ist das Netzwerk bereits nach kurzer Zeit nicht mehr klebrig, d.h. "tack-free".

Zudem lässt sich die erfindungsgemäße Verbindung leicht mit einem Präpolymeren vermischen, da sie bei 23 °C eine Viskosität von weniger als 10.000 mPas aufweist. Auf diese Weise kann die Verbindung auch in 2-komponentigen Sprühsystemen verwendet werden, in denen die einzelnen Komponenten erst in einer Mischdüse während des Ausbringens miteinander vermischt werden.

Im Rahmen des vorgenannten Verfahrens ist vorgesehen, dass die H-funktionelle Starterverbindung wenigstens ein Zerewitinoff-aktives H-Atom trägt. Unter einem Zerewitinoff-aktiven H-Atom wird im Rahmen der vorliegenden Erfindung ein azides H-Atom oder "aktives" H-Atom verstanden. Ein solches kann in an sich bekannter Weise durch eine Reaktivität mit einem entsprechenden Grignard-Reagenz identifiziert werden. Die Menge an Zerewitinoff-aktiven H-Atomen wird typischerweise über die Methanfreisetzung gemessen, die bei einer Reaktion der zu überprüfenden Substanz mit Methylmagnesiumbromid ($CH_3$-MgBr) gemäß der folgenden Reaktionsgleichung (Formel 1) frei wird:

$$CH_3\text{-MgBr} + ROH \rightarrow CH_4 + Mg\,(OR)Br \qquad (1)$$

Zerewitinoff-aktive H-Atome stammen typischerweise von C-H aziden organischen Gruppen, -OH, -SH, $-NH_2$ oder -NHR mit R als organischem Rest sowie -COOH.

Neben den bevorzugt zu verwendenden hydroxyfunktionellen Startern können auch aminofunktionelle Starter eingesetzt werden.

Beispiele für hydroxyfunktionelle Starterverbindungen sind Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, sowie methylolgruppenhaltige Kondensate aus Formaldehyd und Phenol oder Harnstoff. Es können auch hochfunktionelle Starterverbindungen auf Basis von hydrierten Stärkehydrolyseprodukten eingesetzt werden. Solche sind beispielsweise in EP 1525244 A1 beschrieben.

Beispiele für aminogruppenhaltige H-funktionelle Starterverbindungen sind Ammoniak, Ethanolamin, Diethanolamin, Triethanolamin, Isopropanolamin, Diisopropanolamin, Ethylendiamin, Hexamethylendiamin, Anilin, die Isomere des Toluidins, die Isomere des Diaminotoluols, die Isomere des Diaminodiphenylmethans sowie bei der Kondensation von Anilin mit Formaldehyd zu Diaminodiphenylmethan anfallende höherkernige Produkte, ferner methylolgruppenhaltige Kondensate aus Formaldehyd und Melamin sowie Mannichbasen.

Außerdem können als Starterverbindungen auch Ringöffnungsprodukte aus cyclischen Carbonsäureanhydriden und Polyolen eingesetzt werden. Beispiele sind Ringöffnungsprodukte aus Phthalsäureanhydrid oder Bernsteinsäureanhydrid einerseits und Ethylenglykol, Diethylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit oder Sorbit andererseits. Daneben ist es auch möglich, ein- oder mehrfunktionelle Carbonsäuren direkt als Starterverbindungen einzusetzen.

Ferner können in dem Prozess auch vorgefertigte Alkylenoxidadditionsprodukte der erwähnten Starterverbindungen, also Polyetherpolyole vorzugsweise mit OH-Zahlen von 5 bis 1000 mg KOH/g, bevorzugt 10 bis 1000 mg KOH/g, als Starterverbindungen eingesetzt, bzw. dem Reaktionsgemisch zugesetzt werden. Auch ist es möglich, im erfindungsgemäßen Prozess Polyesterpolyole vorzugsweise mit OH-Zahlen im Bereich von 6 bis 800 mg KOH/g als Co-Starter einzusetzen. Hierfür geeignete Polyesterpolyole können beispielsweise aus organischen Dicarbonsäuren mit 2 bis 12 Kohlenstoffatomen und mehrwertigen Alkoholen, vorzugsweise Diolen, mit 2 bis 12 Kohlenstoffatomen, vorzugsweise 2 bis 6 Kohlenstoffatomen nach bekannten Verfahren hergestellt werden.

Des Weiteren können als H-funktionelle Startersubstanzen Polycarbonatpolyole, Polyestercarbonatpolyole oder Polyethercarbonatpolyole, bevorzugt Polycarbonatdiole, Polyestercarbonatdiole oder Polyethercarbonatdiole vorzugsweise jeweils mit OH-Zahlen im Bereich von 6 bis 800 mg KOH/g, als Starter oder Co-Starter verwendet werden. Diese werden beispielsweise durch Umsetzung von Phosgen, Dimethylcarbonat, Diethylcarbonat oder Diphenylcarbonat mit di- oder höherfunktionellen Alkoholen oder Polyesterpolyolen oder Polyetherpolyolen hergestellt.

In Schritt a) des erfindungsgemäßen Verfahrens dienen bevorzugt aminogruppenfreie H-funktionelle Starterverbindungen mit Hydroxygruppen als Träger der aktiven Wasserstoffe, wie beispielsweise Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, methylolgruppenhaltige Kondensate aus Formaldehyd und Phenol und hydrierte Stärkehydrolyseprodukte. Es können auch Gemische verschiedener H-funktioneller Starterverbindungen eingesetzt werden. Ist im Folgenden von einer H-funktionellen Starterverbindung die Rede, so sind damit grundsätzlich auch Gemische H-funktioneller Starterverbindungen gemeint, sofern dies nicht ausdrücklich ausgeschlossen wird.

Für das im Rahmen des erfindungsgemäßen Verfahrens eingesetzte ungesättigte zyklische Carbonsäureanhydrid kommen sämtliche dem Fachmann als solche bekannten Verbindungen in Frage. Dies sind beispielsweise ungesättigte zyklische Dicarbonsäureanhydride wie Maleinsäureanhydrid, Tetrahydrophthalsäureanhydrid, insbesondere 3,4,5,6-Tetrahydrophthalsäureanhydrid sowie Kombinationen hiervon.

Werden mehrere ungesättigte cyclische Carbonsäureanhydride verwendet, so können diese ebenfalls einzeln, im Gemisch oder blockweise dosiert werden. Es ist außerdem möglich, das cyclische Carbonsäureanhydrid oder die cyclischen Carbonsäureanhydride dem Reaktionsgemisch parallel mit dem / den Alkylenoxid(en) oder als separater Block, ohne gleichzeitige Alkylenoxiddosierung, zuzuführen. Ist im Folgenden von einem ungesättigten cyclischen Carbonsäureanhydrid die Rede, so sind damit grundsätzlich auch Gemische ungesättigter cyclischer Carbonsäureanhydride gemeint, sofern nicht ausdrücklich spezifiziert.

Als erfindungsgemäß verwendbare Alkylenoxidverbindung können solche Vertreter ausgewählt sein, die 2 bis 24 Kohlenstoffatome aufweisen, insbesondere 2 bis 12 Kohlenstoffatome, weiter bevorzugt 2 bis 6 Kohlenstoffatome, sowie die Kombination unterschiedlicher Alkylenoxidverbindungen der vorgenannten Art. Bei den Alkylenoxiden mit 2 bis 24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder

mehrfach epoxidierte Fette als Mono-, Di- und Triglyceride, epoxidierte Fettsäuren, $C_1$-$C_{24}$-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyloxysilane wie beispielsweise 3-Glycidyloxypropyltrimethoxysilan, 3-Glycidyloxypropyltriethoxysilan, 3-Glycidyloxypropyltripropoxysilan, 3-Glycidyloxypropyl-methyldimethoxysilan, 3-Glycidyloxypropyl-ethyldiethoxysilan und 3-Glycidyloxypropyltriisopropoxysilan.

Als Alkylenoxide werden bevorzugt Ethylenoxid und/oder Propylenoxid eingesetzt. Besonders bevorzugt wird Ethylenoxid in Anteilen von 40 Gew.-% oder mehr, bevorzugt in Anteilen von 40 bis 90 Gew.-%, bezogen auf die Gesamtmasse der zu dosierenden Alkylenoxide, verwendet. Die Alkylenoxide können einzeln, im Gemisch oder blockweise dosiert werden. Ist im Folgenden von einem Alkylenoxid oder einer Alkylenoxidverbindung die Rede, so sind damit grundsätzlich auch Gemische von Alkylenoxiden oder Alkylenoxidverbindungen oder die blockweise Dosierung von unterschiedlichen Alkylenoxiden bzw. Alkylenoxidverbindungen gemeint, sofern nicht ausdrücklich spezifiziert.

Bei dem erfindungsgemäßen Verfahren kann ferner vorgesehen sein, dass das Stoffmengenverhältnis zwischen Carbonsäureanhydrid und der Zahl der Zerewitinoffaktiven H-Atome der Starterverbindung so ausgewählt ist, dass möglichst alle Zerewitinoffaktiven H-Atome umgesetzt werden. Für diese stöchiometrische Umsetzung kann das Stoffmengenverhältnis zwischen dem Carbonsäureanhydrid und der Zahl der Zerewitinoffaktiven H-Atome der H-funktionellen Starterverbindung etwa 1 : 1 bis 1,5 : 1 betragen, insbesondere 1 : 1 bis 1,2 : 1.

Die erfindungsgemäßen Verfahren sind im Übrigen nicht auf die Verwendung der vorgenannten Monomere beschränkt. So ist es beispielsweise möglich, dass wenigstens ein Co-Monomer umgesetzt wird, welches insbesondere ausgewählt ist aus Lactonen, Lactiden, gesättigten bzw. aromatischen cyclischen Carbonsäureanhydriden, cyclischen Carbonaten und/ oder Kohlendioxid. Auf diese Weise kann das Eigenschaftsprofil des erhaltenen Hydroxy-Aminopolymers weiter modifiziert werden, beispielsweise in Bezug auf seine Reaktivität gegenüber Isocyanatgruppen, seine Polarität sowie andere chemische oder physikalische Eigenschaften des Hydroxy-Aminopolymers bzw. dessen Umsetzungsprodukts mit einem Polyisocyanat. Dieses Co-Monomer wird vorzugsweise im Schritt a) des erfindungsgemäßen Verfahrens zugesetzt.

Im Rahmen des erfindungsgemäßen Verfahrens ist u.a. vorgesehen, dass ein primäres Amin oder Ammoniak an die Doppelbindung des Hydroxylgruppen-tragenden Präpolymers addiert wird. Geeignete Amine sind beispielsweise Ammoniak, aliphatische, cycloaliphatische und/oder araliphatische Monoamine mit einer primären Aminogruppe wie beispielsweise Methylamin, Ethylamin, Diethylamin, 1-Aminopropan, 2-Aminopropan, 1-Aminobutan, 2-Aminobutan, Isobutylamin, 1-Aminohexan, 2-Ethyl-1-Aminohexan, Dodecylamin, Octadecyl-amin, Cyclohexylamin und Benzylamin; aliphatische, cycloaliphatische und/oder araliphatische Diamine mit einer primären Aminogruppe und einer sekundären Aminogruppe, wobei die sekundäre Aminogruppe auch Teil eines Ringsystems sein kann, wie beispielsweise N-Methylethylendiamin, N-Methylpropylendiamin, N-(2-Aminoethyl)-piperazin und 3-Amino-1,2,4-triazol; aliphatische, cycloaliphatische und/ oder heterocyclische Diamine mit einer primären und einer tertiären Aminogruppe und ggf. einer sekundären Aminogruppe wie beispielsweise N,N-dimethylethylendiamin, N,N-dimethyl-1,3-diaminopropan, N,N-dimethyl-1,8-diaminooctan, N,N-dimethyl-1,4-diaminocyclohexan und aliphatische Amine mit zwei primären und mindestens einer sekundären Aminogruppe, wie z. B. Diethylentriamin, Triethylentetramin, Tetraethylenpentamin und Bis-(3-aminopropyl)-amin. Des Weiteren sind Amine, welche neben der primären Aminogruppe auch Hydroxygruppen enthalten, wie beispielsweise Ethanolamin oder Isopropanolamin, für das erfindungsgemäße Verfahren geeignet.

Ebenfalls geeignet sind (cyclo)aliphatische Diamine. Hierbei handelt es sich um Verbindungen mit zwei primären Aminogruppen mit der allgemeinen Formel $NH_2$-R-$NH_2$, in der R für einen aliphatischen oder cycloaliphatischen Rest mit 2 bis 21, vorzugsweise 2 bis 15 und besonders bevorzugt 2 bis 10 Kohlenstoffatomen steht. Beispielhaft zu nennen sind Ethylendiamin, 1,2- und 1,3-Propylendiamin, 1,4-Diamino-butan, 1,6-Diaminohexan, 2,2,4- und 2,4,4-Trimethyl-1,6-diaminohexan, 1,4-Diaminocyclohexan, 1,5-Diamino-2-methylpentan, 5-Amino-1-aminomethyl-1,3,3-trimethylcyclohexan (Isophorondiamin), Bis-(4-aminocyclohexyl)-methan, Bis-(4-amino-3-methylcyclohexyl)-methan, 1-Amino-1-methyl-3(4)-aminomethylcyclohexan, Bis-(4-Amino-3,5-diethylcyclohexyl)-methan, Bis-aminomethyl-hexahydro-4,7-methano-indan, 2,3-, 2,4- und 2,6-Diamino-1-methylcyclohexan bzw. Gemische dieser Diamine. Die genannten Mono- und Oligoamine können natürlich auch als Mischung eingesetzt werden. Ist im Folgenden von einem zu addierenden Amin die Rede, so sind damit grundsätzlich auch Gemische zu addierender Amine gemeint, sofern nicht ausdrücklich spezifiziert.

Das molare Verhältnis von primären Aminogruppen zu additionsfähigen Doppelbindungen beträgt vorzugsweise 0,01 : 1 bis 1,1 : 1, bevorzugt 0,1 : 1 bis 1,1 : 1, besonders bevorzugt 0,5 : 1 bis 1,1 : 1 und ganz besonders bevorzugt 1 : 1 bis 1,1 : 1. Die Reaktion kann katalysiert oder unkatalysiert durchgeführt werden. Geeignete Katalysatoren sind beispielsweise Kupferacetat, Zinnchlorid oder Essigsäure. Bevorzugt erfolgt die Addition der Amine ohne Katalysatorzusatz. Ein für diesen Schritt geeigneter Reaktionstemperaturbereich ist beispielsweise der von 0 °C bis 150 °C, bevorzugt von 10 °C bis 100 °C und besonders bevorzugt 20 °C bis 80 °C.

Nach einer bevorzugten Ausführungsform des erfindungsgemäßen Verfahrens wird das Stoffmengenverhältnis zwischen der Alkylenoxidverbindung und dem Carbonsäureanhydrid auf wenigstens 1 : 1, vorzugsweise auf wenigstens

2 : 1 eingestellt, besonders bevorzugt wenigstens auf 2,5 : 1. Bei dieser Ausführungsform des erfindungsgemäßen Verfahrens ist es möglich, Hydroxy-Aminopolymere mit einem mittleren Abstand von mehr als sieben kovalenten Bindungslängen zwischen der Aminfunktionalität und der Hydroxylgruppe zu synthetisieren.

In weiterer Ausgestaltung des erfindungsgemäßen Verfahrens beträgt das Verhältnis der addierten Aminogruppen zu den Hydroxylgruppen im Hydroxy-Aminopolymer 0,8 bis 2,5, insbesondere 0,9 bis 2,0, vorzugsweise 0,95 bis 1,8. Hierdurch kann die Vernetzungsfähigkeit des Hydroxy-Aminopolymers bei Umsetzung mit einem polyfunktionellen NCO-Präpolymer noch weiter erhöht werden.

Was die OH-Funktionalität des Hydroxy-Aminopolymers betrifft, so kann diese in bevorzugter Weise 1,5 bis 6 betragen, insbesondere 1,7 bis 4, besonders bevorzugt 1,8 bis 3.

Im Rahmen des erfindungsgemäßen Verfahrens ist vorgesehen, dass eine wenigstens ein Zerewitinoff-aktives H-Atom tragende H-funktionelle Starterverbindung eingesetzt wird. Diese Verbindung kann in bevorzugter Weise 1 bis 35 Zerewitinoff-aktive H-Atome aufweisen, insbesondere 1 bis 8.

Die Molmasse der H-funktionellen Starterverbindung kann über weite Bereiche variiert werden. So kann die H-funktionelle Starterverbindung beispielsweise eine zahlenmittlere Molmasse von 17 bis 10000 g/mol aufweisen. Mit anderen Worten ist im Rahmen der vorliegenden Erfindung also vorgesehen, dass von einer monomeren oder kurzkettigen Starterverbindung, beispielsweise Ammoniak, oder aber auch von einer polymeren Starterverbindung ausgegangen werden kann.

<u>Schritt a) erste Verfahrensalternative:</u>

Es kann gemäß einer ersten Alternative die H-funktionelle Starterverbindung zunächst mit einer ersten Menge der Alkylenoxidverbindung und anschließend mit dem ungesättigten cyclischen Carbonsäureanhydrid und einer weiteren Menge der Alkylenoxidverbindung umgesetzt werden. In diesem Fall wird also die Kettenlänge der H-funktionellen Starterverbindung zunächst vergrößert ehe die Umsetzung mit dem ungesättigten cyclischen Carbonsäureanhydrid und einer weiteren Menge an Alkylenoxidverbindung erfolgt. Die H-funktionelle Starterverbindung behält bei dieser Erhöhung der Kettenlänge die Zahl ihrer Zerewitinoff-aktiven H-Atome weitestgehend bei, da zunächst nur Oxyalkyleneinheiten an die ursprüngliche H-funktionelle Starterverbindung addiert werden, wobei eine H-funktionelle Starterverbindung mit einem höheren Molekulargewicht erhalten wird.

Diese Verfahrensweise ist besonders geeignet, wenn von einer H-funktionellen Starterverbindung mit einer zahlenmittleren Molmasse von 17 bis 1200 g/mol ausgegangen wird, insbesondere von 62 bis 1000 g/mol. Diese H-funktionelle Starterverbindung kann dann durch Addition der Alkylenoxidverbindung beispielsweise auf eine zahlenmittlere Molmasse von 200 bis 20000 g/mol aufgebaut werden, bevorzugt von 600 bis 10000 g/mol. Dieses Verfahren ist vorteilhaft, weil damit Strukturen erzeugt werden können, bei denen der Abstand zwischen der Aminfunktionalität und der Hydroxylgruppe mehr als 6 oder 7 kovalente Bindungslängen betragen kann.

In weiterer Ausgestaltung des erfindungsgemäßen Verfahrens wird die Umsetzung der H-funktionellen Starterverbindung mit dem ungesättigten cyclischen Carbonsäureanhydrid und/ oder die Addition der Alkylenoxidverbindung unter Einsatz eines Doppelmetallcyanid-Katalysators (DMC-Katalysator) durchgeführt, wobei der DMC-Katalysator insbesondere Zinkhexacyanocobaltat (III), Zinkhexacyanoiridat (III), Zinkhexacyanoferrat (III) und Cobalt(II) hexacyanocobaltat (III) enthält.

Das Verfahren kann z. B. auch derart ausgestaltet sein, dass nach Abreaktion einer dosierten Alkylenoxidverbindung ungesättigtes cyclisches Carbonsäureanhydrid zugeführt wird, beispielsweise etwa 1 Mol Carbonsäureanhydrid pro Mol vorhandener OH-Gruppen. Danach wird nochmals eine gewünschte Menge an Alkylenoxidverbindungen addiert und das Hydroxylgruppen tragende Präpolymer zu erhalten. Die vorgenannte Reaktionsfolge lässt sich auch ein- oder mehrfach wiederholen, sodass eine gewünschte Zahl, insbesondere mehr als eine Doppelbindung pro Zerewitinoff-aktivem H-Atom in das Präpolymer eingebaut werden kann. So können beispielsweise 2 oder mehr, insbesondere 3 oder mehr Aminfunktionalitäten pro Zerewitinoff-aktivem H-Atom durch Addition an die Doppelbindungen eingeführt werden. Natürlich lassen sich die Doppelbindungen in das Präpolymer auch durch parallele Dosierung von einer oder mehreren Alkylenoxidverbindungen und einem oder mehreren ungesättigten cyclischen Carbonsäureanhydriden zu der einen oder mehreren Zerewitinoff-aktive H-Atome tragenden Starterverbindungen einführen. Diese Paralleldosierung von einer oder mehreren Alkylenoxidverbindungen und einem oder mehreren ungesättigten cyclischen Carbonsäureanhydriden kann von Anfang an erfolgen oder erst nachdem ein reiner Alkylenoxidblock zu der Zerewitinoff-aktive H-Atome tragenden Starterverbindung dosiert wurde. Die Verteilung der Doppelbindungen auf die Polymerketten des Präpolymers erfolgt bei Parallelosierung von Alkylenoxidverbindung(en) und ungesättigtem cyclischen Carbonsäureanhydrid nach statistischen Gesetzmäßigkeiten, insbesondere werden die Blöcke der auf Alkylenoxidbausteinen basierenden Polyetherketten einer breiteren Längenverteilung unterliegen.

Bei dem erfindungsgemäßen Verfahren ist vorgesehen, dass im Schritt a), also bei der Umsetzung der H-funktionellen Starterverbindung mit dem ungesättigten zyklischen Carbonsäureanhydrid und/ oder der Addition der Alkylenoxidverbindung ein Doppelmetallcyanid-Katalysator (DMC-Katalysator) eingesetzt wird. Dabei können auch Mischungen verschiedener DMC-Katalysatoren verwendet werden.

Geeignete DMC-Katalysatoren sind im Prinzip aus dem Stand der Technik bekannt und werden beispielsweise in US 3,404,109 A1, US 3,829,505 A1, US 3,941,849 A1 und US 5,158,922 A1 offenbart.

DMC-Katalysatoren, die z.B. in US 5,470,813 A1, EP 700949 A1, EP 743 093 A1, EP 761 708 A1, WO 97/40086 A1, WO 98/16310 A1 und WO 00/47649 A1 beschrieben sind, besitzen eine sehr hohe Aktivität in der Polymerisation von Alkylenoxiden und ggf. der Copolymerisation von Alkylenoxiden und ungesättigten cyclischen Carbonsäureanhydriden und ermöglichen die Herstellung von Polyetherpolyolen bei sehr geringen Katalysatorkonzentrationen (25 ppm oder weniger), so dass eine Abtrennung des Katalysators aus dem fertigen Produkt im Allgemeinen nicht mehr erforderlich ist. Ein typisches Beispiel sind die in EP 700 949 A1 beschriebenen hochaktiven DMC-Katalysatoren, die neben einer Doppelmetallcyanid-Verbindung wie Zinkhexacyanocobaltat(III) und einem organischen Komplexliganden wie tert.-Butanol noch einen Polyether mit einem zahlenmittlerem Molekulargewicht größer als 500 g/mol enthalten. Es ist auch möglich, die in EP Anmeldenummer 10163170.3 offenbarten alkalischen DMC-Katalysatoren einzusetzen.

Zur Herstellung der Doppelmetallcyanid-Verbindungen geeignete cyanidfreie Metallsalze besitzen bevorzugt die allgemeine Formel (III),

$$M(X)_n \qquad (III)$$

wobei

M ausgewählt ist aus den Metallkationen $Zn^{2+}$, $Fe^{2+}$, $Ni^{2+}$, $Mn^{2+}$, $Co^{2+}$, $Sr^{2+}$, $Sn^{2+}$, $Pb^{2+}$ und, $Cu^{2+}$, bevorzugt ist M $Zn^{2+}$, $Fe^{2+}$, $Co^{2+}$ oder $Ni^{2+}$,

X für ein oder mehrere (d.h.verschiedene) Anionen steht, welches vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

n ist 1, wenn X = Sulfat, Carbonat oder Oxalat ist und

n ist 2, wenn X = Halogenid, Hydroxid, Cyanat, Thiocyanat, Isocyanat, Isothiocyanat oder Nitrat ist.

Weiter geeignete cyanidfreie Metallsalze besitzen die allgemeine Formel (IV),

$$M_r(X)_3 \qquad (IV)$$

wobei

M ausgewählt ist aus den Metallkationen $Fe^{3+}$, $Al^{3+}$ und $Cr^{3+}$,

X steht für einen oder unterschiedliche Anionentypen, wobei das Anion vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

r ist 2, wenn X = Sulfat, Carbonat oder Oxalate ist und

r ist 1, wenn X = Halogenid, Hydroxid, Cyanat, Thiocyanat, Isocyanat, Isothiocyanat, Carboxylat oder Nitrat ist.

Andere geeignete cyanidfreie Metallsalze besitzen die allgemeine Formel (V),

$$M(X)_s \qquad (V)$$

wobei

M ausgewählt ist aus den Metallkationen $Mo^{4+}$, $V^{4+}$ und $W^{4+}$

X steht für einen oder unterschiedliche Anionentypen, wobei das Anion vorzugsweise ausgewählt ist aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

s ist 2, wenn X = Sulfat, Carbonat oder Oxalat ist und

s ist 4, wenn X = Halogenid, Hydroxid, Cyanat, Thiocyanat, Isocyanat, Isothiocyanat, Carboxylat oder Nitrat ist.

Ebenfalls geeignete cyanidfreie Metallsalze besitzen die allgemeine Formel (VI),

$M(X)_t$ (VI)

wobei

M ausgewählt ist aus den Metallkationen $Mo^{6+}$ und $W^{6+}$

X steht für einen oder unterschiedliche Anionentypen, wobei das Anion vorzugsweise ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

t ist 3, wenn X = Sulfat, Carbonat oder Oxalat ist und

t ist 6, wenn X = Halogenid, Hydroxid, Cyanat, Thiocyanat, Isocyanat, Isothiocyanat, Carboxylat oder Nitrat ist,

Beispiele geeigneter cyanidfreier Metallsalze sind Zinkchlorid, Zinkbromid, Zinkjodid, Zinkacetat, Zinkacetylacetonat, Zinkbenzoat, Zinknitrat, Eisen(II)sulfat, Eisen(II)bromid, Eisen(II)chlorid, Cobalt(II)chlorid, Cobalt(II)thiocyanat, Nickel(II)chlorid und Nickel-(II)nitrat. Es können auch Mischungen verschiedener Metallsalze eingesetzt werden.

Zur Herstellung der Doppelmetallcyanid-Verbindungen geeignete Metallcyanidsalze besitzen bevorzugt die allgemeine Formel (VII)

$(Y)_aM'(CN)_b(A)$ (VII)

wobei

M' ausgewählt ist aus einem oder mehreren Metallkationen der Gruppe bestehend aus Fe(II), Fe(III), Co(II), Co(III), Cr(II), Cr(III), Mn(II), Mn(III), Ir(III), Ni(II), Rh(III), Ru(II), V(IV) und V(V), bevorzugt ist M' ein oder mehrere Metallkationen der Gruppe bestehend aus Co(II), Co(III), Fe(II), Fe(III), Cr(III), Ir(III) und Ni(II),

Y ausgewählt ist aus einem oder mehreren Metallkationen der Gruppe bestehend aus Alkalimetall (d.h. $Li^+$, $Na^+$, $K^+$, $Rb^+$, $Cs^+$) und Erdalkalimetall (d.h. $Be^{2+}$, $Ca^{2+}$, $Mg^{2+}$, $Sr^{2+}$, $Ba^{2+}$),

A ausgewählt ist aus einem oder mehreren Anionen der Gruppe bestehend aus Halogeniden (d..h. Fluorid, Chlorid, Bromid, Iodid), Hydroxid, Sulfat, Carbonat, Cyanat, Thiocyanat, Isocyanat, Isothiocyanat, Carboxylat, Oxalat oder Nitrat und

a, b und c sind ganzzahlige Zahlen, wobei die Werte für a, b und c so gewählt sind, dass die Elektroneutralität des Metallcyanidsalzes gegeben ist; a ist vorzugsweise 1, 2, 3 oder 4; b ist vorzugsweise 4, 5 oder 6; c besitzt bevorzugt den Wert 0.

Beispiele geeigneter Metallcyanidsalze sind Kaliumhexacyanocobaltat(III), Kaliumhexacyanoferrat(II), Kaliumhexacyanoferrat(III), Calciumhexacyanocobaltat(III) und Lithiumhexacyanocobaltat(III).

Bevorzugte Doppelmetallcyanid-Verbindungen, die in den erfindungsgemäßen DMC-Katalysatoren enthalten sind, sind Verbindungen der allgemeinen Formel (VIII)

$M_x[M'_{x'},(CN)_y]_z$ (VIII),

worin M wie in Formel (III) bis (VI) und

M' wie in Formel (VII) definiert ist, und

x, x', y und z sind ganzzahlig und so gewählt, dass die Elektronenneutralität der Doppelmetallcyanidverbindung gegeben ist.

Vorzugsweise ist
$x = 3$, $x' = 1$, $y = 6$ und $z = 2$,
M = Zn(II), Fe(II), Co(II) oder Ni(II) und
M' = Co(III), Fe(III), Cr(III) oder Ir(III).

Beispiele bevorzugt eingesetzter Doppelmetallcyanidverbindungen sind Zinkhexacyanocobaltat(III), Zinkhexacyanoiridat(III), Zinkhexacyanoferrat(III) und Cobalt(II)hexacyanocobaltat(III). Weitere Beispiele geeigneter Doppelmetallcyanid-Verbindungen sind z.B. US 5,158,922 A1 zu entnehmen. Besonders bevorzugt verwendet wird Zinkhexacyanocobaltat(III).

Die bei der Herstellung der DMC-Katalysatoren zugesetzten organischen Komplexliganden sind beispielsweise in US-A 5,158,922 A1, US 3,404,109 A1, US 3,829,505 A1, US 3,941,849 A1, EP 700949 A1, EP 761708 A1, JP 4145123 A1, US 5,470,813 A1, EP 743 093 A1 und WO 97/40086 A1 offenbart. Beispielsweise werden als organische Komplexliganden wasserlösliche, organische Verbindungen mit Heteroatomen, wie Sauerstoff, Stickstoff, Phosphor oder Schwefel, die mit der Doppelmetallcyanid - Verbindung Komplexe bilden können, eingesetzt. Bevorzugte organische Komplexliganden sind Alkohole, Aldehyde, Ketone, Ether, Ester, Amide, Harnstoffe, Nitrile, Sulfide und deren Mischungen. Besonders bevorzugte organische Komplexliganden sind aliphatische Ether (wie Dimethoxyethan), wasserlösliche aliphatische Alkohole (wie Ethanol, Isopropanol, n-Butanol, iso-Butanol, sek.-Butanol, tert-Butanol, 2-Methyl-3-buten-2-ol und 2-Methyl-3-butin-2-ol), Verbindungen, die sowohl aliphatische oder cycloaliphatische Ethergruppen wie auch aliphatische Hydroxylgruppen enthalten (wie z.B. Ethylenglykol-mono-tert.-butylether, Diethylenglykol-mono-tert.-butylether, Tripropylenglykol-mono-methylether und 3-Methyl-3-oxetan-methanol). Höchst bevorzugte organische Komplexliganden sind ausgewählt aus einer oder mehrerer Verbindungen der Gruppe bestehend aus Dimethoxyethan, tert-Butanol, 2-Methyl-3-buten-2-ol, 2-Methyl-3-butin-2-ol, Ethylenglykol-mono-tert.-butylether und 3-Methyl-3-oxetan-methanol.

Optional werden bei der Herstellung der erfindungsgemäß bevorzugten DMC-Katalysatoren eine oder mehrere komplexbildende Komponente(n) aus den Verbindungsklassen der Polyether, Polyester, Polycarbonate, Polyalkylenglykolsorbitanester, Polyalkylenglykolglycidylether, Polyacrylamid, Poly(acrylamid-co-acrylsäure), Polyacrylsäure, Poly(acrylsäure-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose und Polyacetale, oder der Glycidylether, Glycoside, Carbonsäureester mehrwertiger Alkohole, Gallensäuren oder deren Salze, Ester oder Amide, Cyclodextrine, Phosphorverbindungen, $\alpha$,$\beta$-ungesättigten Carbonsäureester oder ionische oberflächen- bzw. grenzflächenaktiven Verbindungen eingesetzt.

Bevorzugt werden bei der Herstellung der erfindungsgemäß bevorzugten DMC-Katalysatoren im ersten Schritt die wässrigen Lösungen des Metallsalzes (z.B. Zinkchlorid), eingesetzt im stöchiometrischen Überschuss (mindestens 50 Mol-%) bezogen auf Metallcyanidsalz, (also mindestens ein molares Verhältnis von cyanidfreiem Metallsalz zu Metallcyanidsalz von 2,25 zu 1,00) und des Metallcyanidsalzes (z.B. Kaliumhexacyanocobaltat) in Gegenwart des organischen Komplexliganden (z.B. tert.-Butanol) umgesetzt, so dass sich eine Suspension bildet, die die Doppelmetallcyanid-Verbindung (z.B. Zinkhexacyanocobaltat), Wasser, überschüssiges cyanidfreies Metallsalz, und den organischen Komplexliganden enthält. Der organische Komplexligand kann dabei in der wässrigen Lösung des cyanidfreien Metallsalzes und/oder des Metallcyanidsalzes vorhanden sein, oder er wird der nach Ausfällung der Doppelmetallcyanid-Verbindung erhaltenen Suspension unmittelbar zugegeben. Es hat sich als vorteilhaft erwiesen, die wässrigen Lösungen des cyanidfreien Metallsalzes und des Metallcyanidsalzes und den organischen Komplexliganden unter starkem Rühren zu vermischen. Optional wird die im ersten Schritt gebildete Suspension anschließend mit einer weiteren komplexbildenden Komponente behandelt. Die komplexbildende Komponente wird dabei bevorzugt in einer Mischung mit Wasser und organischem Komplexliganden eingesetzt. Ein bevorzugtes Verfahren zur Durchführung des ersten Schrittes (d.h. der Herstellung der Suspension) erfolgt unter Einsatz einer Mischdüse, besonders bevorzugt unter Einsatz eines Strahldispergators wie in WO 01/39883 A1 beschrieben.

Im zweiten Schritt erfolgt die Isolierung des Feststoffs (d.h. die Vorstufe des erfindungsgemäßen Katalysators) aus der Suspension durch bekannte Techniken, wie Zentrifugation oder Filtration.

In einer bevorzugten Ausführungsvariante zur Herstellung des Katalysators wird der isolierte Feststoff anschließend in einem dritten Verfahrensschritt mit einer wässrigen Lösung des organischen Komplexliganden gewaschen (z.B. durch Resuspendieren und anschließende erneute Isolierung durch Filtration oder Zentrifugation). Auf diese Weise können zum Beispiel wasserlösliche Nebenprodukte, wie Kaliumchlorid, aus dem erfindungsgemäßen Katalysator entfernt werden. Bevorzugt liegt die Menge des organischen Komplexliganden in der wässrigen Waschlösung zwischen 40 und 80 Gew.-%, bezogen auf die Gesamtlösung.

Optional wird im dritten Schritt der wässrigen Waschlösung eine weitere komplexbildende Komponente, bevorzugt im Bereich zwischen 0,5 und 5 Gew.-%, bezogen auf die Gesamtlösung, zugefügt.

Außerdem ist es vorteilhaft, den isolierten Feststoff mehr als einmal zu waschen. Hierzu kann z.B. der erste

Waschvorgang wiederholt werden. Bevorzugt ist es aber, für weitere Waschvorgänge nicht wässrige Lösungen zu verwenden, z.B. eine Mischung aus organischem Komplexliganden und weiterer komplexbildender Komponente.

Der isolierte und gegebenenfalls gewaschene Feststoff wird anschließend, gegebenenfalls nach Pulverisierung, bei Temperaturen von im allgemeinen 20 - 100 °C und bei Drücken von im allgemeinen 0,1 mbar bis Normaldruck (1013 mbar) getrocknet.

Ein bevorzugtes Verfahren zur Isolierung der erfindungsgemäßen DMC-Katalysatoren aus der Suspension durch Filtration, Filterkuchenwäsche und Trocknung wird in WO 01/80994 A1 beschrieben.

Die Konzentration an in Schritt a) eingesetztem DMC-Katalysator beträgt 5 bis 1000 ppm, bevorzugt 10 bis 900 ppm und besonders bevorzugt 20 bis 800 ppm, bezogen auf die Menge des herzustellenden, Hydroxylgruppen tragenden Präpolymers. Je nach Anforderungsprofil der der Aminaddition nachgeschalteten Anwendung kann der DMC-Katalysator im Produkt belassen oder (teilweise) abgetrennt werden Die (teilweise) Abtrennung des DMC-Katalysators kann beispielsweise durch Behandlung mit Adsorbentien erfolgen. Verfahren zur Abtrennung von DMC-Katalysatoren sind beispielsweise beschrieben in US 4,987,271 A1, DE 313 22 58 A1, EP 406 440 A1, US 5,391,722 A1, US 5,099,075 A1, US 4,721,818 A1, US 4,877,906 A1 und EP 385 619 A1.

Der H-funktionellen Starterverbindung können ggf. vor Inkontaktbringen mit dem DMC-Katalysator geringe Mengen einer anorganischen Mineralsäure, bevorzugt Phosphorsäure, zugesetzt werden, um etwaige Basenspuren in der H-funktionellen Starterverbindung zu neutralisieren.

Wird das erfindungsgemäße Verfahren unter Verwendung von DoppelmetallcyanidKatalysatoren durchgeführt, so ist es weiterhin von Vorteil, zunächst die H-funktionelle Starterverbindung und den Katalysator vorzulegen, eine Teilmenge der AlkylenoxidVerbindung sowie gegebenenfalls weiterer Co-Monomere zuzudosieren und erst anschließend das ungesättigte zyklische Carbonsäureanhydrid zuzugeben. Auf diese Weise kann ausgehend von der H-funktionellen Starterverbindung zunächst ein doppelbindungsfreies Polymergerüst aufgebaut werden. Zu diesem Zweck können sämtliche der vorgenannten Alkylenoxidverbindungen bzw. ggf. zusätzliche Co-Monomere verwendet werden. Das ungesättigte zyklische Carbonsäureanhydrid wird typischerweise dann der Reaktionsmischung zugeführt, wenn die vorgenannte Additionsreaktion der Alkylenoxidverbindung an die H-funktionelle Starterverbindung abgeschlossen ist.

Nachdem das ungesättigte zyklische Carbonsäureanhydrid zugegeben wurde, wird anschließend nochmals die Alkylenoxidverbindung zugegeben sowie gegebenenfalls weiteres Co-Monomer. Hierbei kann durch Wahl der Stoffmengen der Alkylenoxidverbindung(en) in Bezug auf die Stoffmenge des zugegebenen ungesättigten cyclischen Carbonsäureanhydrids der Abstand zwischen der Aminfunktionalität und der Hydroxylgruppe wie vorgenannt beschrieben eingestellt werden, wobei insbesondere mehr als 1 mol der Alkylenoxidverbindung pro mol Zerewitinoff-aktivem Wasserstoff zugegeben wird. Auch durch Zugabe weiteren Co-Monomers kann auf den Abstand dieser beiden Funktionalitäten voneinander eingewirkt werden. Wie bereits oben erwähnt, kann anschließend nochmals Carbonsäureanhydrid und nach dessen Abreaktion weitere Alkylenoxidverbindung hinzugegeben werden, um die Möglichkeit zum Einbau von mehr als einer Aminfunktion pro Zerewitinoff-aktivem H-Atom vorzusehen.

Im Folgenden wird Schritt a) des erfindungsgemäßen Verfahrens gemäß dieser Variante detailliert beschrieben, wobei die vorliegende Erfindung nicht auf die folgende Darstellung beschränkt ist:

> In einer Ausführungsform des erfindungsgemäßen Verfahrens wird die H-funktionelle Verbindung mit dem DMC-Katalysator zunächst in einem Reaktor / Reaktorsystem vorgelegt. Der H-funktionellen Verbindung können ggf. vor Inkontaktbringen mit dem DMC-Katalysator geringe Mengen einer anorganischen Mineralsäure, bevorzugt Phosphorsäure, zugesetzt werden, um etwaige Basenspuren in der H-funktionellen Starterverbindung zu neutralisieren, bzw. um den Produktionsprozess generell stabiler zu gestalten.

Nach Aufheizen auf Temperaturen von 50 bis 160 °C, insbesondere 60 bis 140 °C, ganz besonders bevorzugt 70 bis 140 °C wird der Reaktorinhalt in einer bevorzugten Verfahrensvariante mit Inertgas über einen Zeitraum bevorzugt 10 bis 60 min. unter Rühren gestrippt. Beim Strippen mit Inertgas werden flüchtige Bestandteile unter Einleiten von Inertgasen in die Flüssigphase bei gleichzeitig angelegtem Vakuum, bei einem absoluten Druck von 5 bis 500 mbar, entfernt. Nach dem Eindosieren von typischerweise 5 bis 20 Gew.-% eines oder mehrerer Alkylenoxide, enthaltend ggf. bereits eine kleine Menge des ungesättigten cyclischen Carbonsäureanhydrids und / oder weiteren Co-Monomers, bezogen auf die Menge an vorgelegter H-funktioneller Verbindung, wird der DMC-Katalysator aktiviert.

Die Zugabe eines oder mehrerer Alkylenoxide und ggf. einer kleinen Menge des ungesättigten cyclischen Carbonsäureanhydrids und / oder weiteren Co-Monomers kann vor, während oder nach dem Aufheizen des Reaktorinhaltes auf Temperaturen von 50 bis 160 °C, bevorzugt 60 bis 140 °C, ganz besonders bevorzugt 70 bis 140 °C geschehen; sie erfolgt bevorzugt nach dem Strippen. Die Aktivierung des Katalysators macht sich durch einen beschleunigten Abfall des Reaktordruckes bemerkbar, wodurch der beginnende Alkylenoxidumsatz / Umsatz des ungesättigten cyclischen Carbonsäurenahydrids angezeigt wird.

Dem Reaktionsgemisch kann sodann die gewünschte Menge Alkylenoxid bzw. Alkylenoxidgemisch, ggf. gemeinsam mit der zu dosierenden Menge an ungesättigtem cyclischen Carbonsäureanhydrid und / oder weiterem Co-Monomer kontinuierlich zugeführt werden, wobei eine Reaktionstemperatur von 20 bis 200 °C, bevorzugt aber von 50 bis 160 °C gewählt wird. Die Reaktionstemperatur ist in den vielen Fällen identisch mit der Aktivierungstemperatur.

Der H-funktionellen Starterverbindung oder dem Reaktionsgemisch kann ferner vor der Zudosierung des ungesättigten zyklischen Carbonsäureanhydrids ein Inhibitor, wie beispielsweise ein Phenolderivat, Phenothiazin oder Vitamin E zugesetzt werden.

Oft erfolgt die Katalysatoraktivierung bereits so schnell, dass die Dosierung einer separaten Menge Alkylenoxid / des ungesättigten cyclischen Carbonsäureanhydrids zur Katalysatoraktivierung entfallen kann und direkt, gegebenenfalls zunächst mit einer reduzierten Dosierrate, mit der kontinuierlichen Dosierung des Alkylenoxids und des ungesättigten cyclischen Carbonsäureanhydrids begonnen werden kann. Auch kann die Reaktionstemperatur während der Alkylenoxiddosierphase / der Dosierung des ungesättigten cyclischen Carbonsäureanhydrids innerhalb der beschriebenen Grenzen variiert werden. Ebenfalls können die Alkylenoxide und das cyclische Carbonsäureanhydrid dem Reaktor auf unterschiedliche Weise zugeführt werden: Möglich ist eine Dosierung in die Gasphase oder direkt in die Flüssigphase, z. B. über ein Tauchrohr oder einen in der Nähe des Reaktorbodens in einer gut durchmischten Zone befindlichen Verteilerring.

Bei DMC-katalysierten Prozessen ist die Dosierung in die Flüssigphase die bevorzugte Variante. Das Alkylenoxid und das ungesättigte cyclische Carbonsäureanhydrid sollten dem Reaktor kontinuierlich derart zugeführt werden, dass die sicherheitstechnischen Druckgrenzen des verwendeten Reaktorsystems nicht überschritten werden. Insbesondere bei der Codosierung von ethylenoxidhaltigen Alkylenoxidgemischen oder reinem Ethylenoxid ist darauf zu achten, dass ein ausreichender Inertgaspartialdruck im Reaktor während der Anfahr- und Dosierphase aufrechterhalten wird. Dieser kann beispielsweise durch Edelgase oder Stickstoff eingestellt werden.

Bei Dosierung in die Flüssigphase sollten die Dosieraggregate selbstleerend ausgelegt sein, beispielsweise durch Anbringen der Dosierbohrungen an der Unterseite des Verteilerrings. Generell sollte durch apparative Maßnahmen, beispielsweise durch die Montage von Rückschlagklappen, ein Rückströmen von Reaktionsmedium in die Dosieraggregate und Eduktvorlagen verhindert werden. Wird ein Alkylenoxid- / Carbonsäureanhydridgemisch dosiert, können die jeweiligen Alkylenoxide und die jeweiligen ungesättigten cyclischen Carbonsäureanhydride dem Reaktor separat oder als Mischung zugeführt werden. Eine Vorvermischung der Alkylenoxide untereinander und mit dem ungesättigten cyclischen Carbonsäureanhydrid kann beispielsweise durch ein in der gemeinsamen Dosierstrecke befindliches Mischaggregat erreicht werden ("inline-blending"). Es hat sich auch bewährt, Alkylenoxide und ggf. das ungesättigte cyclische Carbonsäureanhydrid pumpendruckseitig in einen beispielsweise über Wärmetauscher geführten Umpumpkreislauf einzeln oder vorgemischt zu dosieren. Für die gute Durchmischung mit dem Reaktionsmedium ist es dann von Vorteil ein hochscherendes Mischaggregat in den Alkylenoxid-/ Carbonsäureanhydrid-/ Reaktionsmediumstrom zu integrieren. Die Temperatur der exothermen ringöffnenden Additionsreaktion wird durch Kühlung auf dem gewünschten Niveau gehalten. Gemäß dem Stand der Technik zur Auslegung von Polymerisationsreaktoren für exotherme Reaktionen (z.B. Ullmann's Encyclopedia of Industrial Chemistry, Vol. B4, pp 167ff, 5th Ed., 1992) erfolgt eine solche Kühlung im Allgemeinen über die Reaktorwand (z.B. Doppelmantel, Halbrohrschlange) sowie mittels weiterer intern im Reaktor und/oder extern im Umpumpkreislauf angeordneter Wärmetauscherflächen, z.B. an Kühlschlangen, Kühlkerzen, Platten- Rohrbündel- oder Mischerwärmetauschern Diese sollten so ausgelegt sein, dass auch zu Beginn der Dosierphase, d. h. bei kleinem Füllstand, effektiv gekühlt werden kann.

Generell sollte in allen Reaktionsphasen durch Auslegung und Einsatz handelsüblicher Rührorgane für eine gute Durchmischung des Reaktorinhaltes gesorgt werden, wobei hier insbesondere ein- oder mehrstufig angeordnete Rührer oder großflächig über die Füllhöhe wirkende Rührertypen geeignet sind (siehe z. B. Handbuch Apparate; Vulkan-Verlag Essen, 1. Aufl. (1990), S.188 - 208). Technisch besonders relevant ist hierbei eine im Mittel über den gesamten Reaktorinhalt eingetragene Mischenergie, die im Allgemeinen im Bereich von 0,2 bis 5 W/l liegt, mit entsprechend höheren lokalen Leistungseinträgen im Bereich der Rührorgane selbst und ggf. bei niedrigen Füllständen. Um eine optimale Rührwirkung zu erzielen, können im Reaktor gemäß allgemeinem Stand der Technik Kombinationen aus Stromstörern (z. B. Flach- oder Rohrstromstörer) und Kühlschlangen (oder Kühlkerzen) angeordnet werden, die sich auch über den Behälterboden erstrecken können. Die Rührleistung des Mischaggregates kann während der Dosierphase auch füllstandsabhängig variiert werden, um in kritischen Reaktionsphasen einen besonders hohen Energieeintrag zu gewährleisten. Beispielsweise kann es vorteilhaft sein, feststoffhaltige Dispersionen, die zu Reaktionsbeginn beispielsweise bei der Verwendung von Saccharose vorliegen können, besonders intensiv zu durchmischen.

Außerdem sollte insbesondere beim Einsatz fester H-funktioneller Starterverbindungen durch die Wahl des Rühraggregates sichergestellt werden, dass eine ausreichende Dispergierung des Feststoffes im Reaktionsgemisch gewährleistet ist. Bevorzugt werden hier bodengängige Rührstufen sowie besonders zur Suspendierung geeignete Rührorgane eingesetzt. Ferner sollte die Rührergeometrie zur Minderung des Aufschäumens von Reaktionsprodukten beitragen. Das Aufschäumen von Reaktionsgemischen kann beispielsweise nach Ende der Dosier- und Nachreaktionsphase beobachtet werden, wenn Restepoxide zusätzlich im Vakuum bei absoluten Drücken im Bereich von 1 bis 500 mbar entfernt werden. Für solche Fälle haben sich Rührorgane als geeignet herausgestellt, die eine kontinuierliche Durchmischung der Flüssigkeitsoberfläche erzielen. Je nach Anforderung weist die Rührwelle ein Bodenlager und gegebenenfalls weitere Stützlager im Behälter auf. Der Antrieb der Rührerwelle kann dabei von oben oder unten erfolgen (mit zentrischer oder exzentrischer Anordnung der Welle).

Alternativ ist es auch möglich, die notwendige Durchmischung ausschließlich über einen Wärmetauscher geführten Umpumpkreislauf zu erzielen oder diesen zusätzlich zum Rühraggregat als weitere Mischkomponente zu betreiben, wobei der Reaktorinhalt nach Bedarf (typischerweise 1 bis 50 Mal pro Stunde) umgepumpt wird.

Für die Durchführung des erfindungsgemäßen Herstellungsverfahrens sind die unterschiedlichsten Reaktortypen geeignet. Vorzugsweise werden zylinderförmige Behälter eingesetzt, welche ein Höhen-/Durchmesserverhältnis von 1:1 bis 10:1 besitzen. Als Reaktorböden kommen beispielsweise Kugel-, Klöpper-, Flach,- oder Konusböden in Frage.

Nach Ende der Dosierung des Alkylenoxids und des ungesättigten cyclischen Carbonsäureanhydrids sowie ggf. weiterer Co-monomere in Schritt a) kann sich eine Nachreaktionsphase anschließen, in der restliches Alkylenoxid / ungesättigtes cyclisches Carbonsäureanhydrid / weiteres Co-Monomer abreagiert. Das Ende dieser Nachreaktionsphase ist erreicht, wenn kein weiterer Druckabfall im Reaktionskessel feststellbar ist. Spuren unreagierter Alkylenoxide / ungesättigter cyclischer Carbonsäureanhydride können nach der Reaktionsphase gegebenenfalls im Vakuum bei einem absoluten Druck von 1 bis 500 mbar oder durch Strippen quantitativ entfernt werden. Durch Strippen werden flüchtige Bestandteile, wie beispielsweise (Rest-)Alkylenoxide, unter Einleiten von Inertgasen oder Wasserdampf in die Flüssigphase bei gleichzeitig angelegtem Vakuum (beispielsweise durch Durchleiten von Inertgas bei einem Absolutdruck von 5 bis 500 mbar) entfernt. Das Entfernen flüchtiger Bestandteile, wie beispielsweise nicht umgesetzter Epoxide, entweder im Vakuum oder durch Strippen, erfolgt bei Temperaturen von 20 bis 200 °C, bevorzugt bei 50 bis 160 °C und vorzugsweise unter Rühren. Solche Strippvorgänge können auch in sog. Strippkolonnen durchgeführt werden, in denen dem Produktstrom ein Inertgas- oder Wasserdampfstrom entgegengeleitet wird. Bevorzugt wird das Strippen mit Inertgasen in Abwesenheit von Wasserdampf durchgeführt. Nach Erreichen von Druckkonstanz bzw. nach Entfernen flüchtiger Bestandteile durch Vakuum und/oder Strippen kann das Produkt aus dem Reaktor abgelassen werden.

Bei Variante A) der ersten Verfahrensalternative von Schritt a) kann die Dosierung des cyclischen Carbonsäureanhydrids so erfolgen, dass die Alkylenoxiddosierung / Dosierung weiterer Comonomere unterbrochen wird, und, ggf. nach einer Nachreaktionsphase, das ungesättigte cyclische Carbonsäureanhydrid dem Reaktor zugeführt wird und nach Zuführung der gewünschten Menge an ungesättigtem cyclischem Carbonsäureanhydrid die Alkylenoxiddosierung / Dosierung weiterer Comonomere wieder aufgenommen wird. Diese Vorgehensweise kann natürlich während eines Reaktionsansatzes auch mehrmals wiederholt werden. Besonders bevorzugt bei dieser Verfahrensweise ist, dass der abschließende Alkylenoxidblock eine Menge von größer 1 mol Alkylenoxid pro mol aktiver H-Atome aus den als Starterverbindungen eingesetzten H-funktionellen Verbindungen umfasst.

Es ist ebenso möglich, das Verhältnis der Dosiergeschwindigkeiten von Alkylenoxiddosierung und der Dosierung des ungesättigten cyclischen Carbonsäureanhydrids während einer parallelen Zudosierung dieser beiden Komponenten gegeneinander kontinuierlich oder stufenweise zu varriieren, indem beispielsweise das Verhältnis des Dosierstroms des ungesättigten cyclischen Carbonsäureanhydrids zu dem des Alkylenoxids / der Alkylenoxide Werte von 0:1 bis 1:0 annimmt.

Ein Charakteristikum von DMC-Katalysatoren ist ihre ausgeprägte Empfindlichkeit gegen hohe Konzentrationen an Hydroxylgruppen, welche beispielsweise durch große Mengen an Startern wie Ethylenglykol, Propylenglykol, Glycerin, Trimethylolpropan, Sorbitol oder Saccharose hervorgerufen werden, und polare Verunreinigungen des Reaktionsgemisches bzw. des Starters oder der Starter. Die DMC-Katalysatoren können dann während der Reaktionsinitiierungsphase nicht in die polymerisationsaktive Form überführt werden. Verunreinigungen können beispielsweise Wasser oder Verbindungen mit einer hohen Zahl in enger Nachbarschaft stehender Hydroxylgruppen wie Kohlenhydrate und Kohlenhydratderivate sein. Auch Substanzen mit in enger Nachbarschaft stehenden Carbonylgruppen bzw. zu Hydroxylgruppen benachbarten Carbonylgruppen wirken sich nachteilig auf die Katalysatoraktivität aus.

Um Starter mit hohen Konzentrationen an OH-Gruppen, bzw. Starter mit als Katalysatorgiften anzusehenden Verunreinigungen dennoch DMC-katalysierten Alkylenoxidadditionsreaktionen unterziehen zu können, sollte die Hydroxylgruppenkonzentration gesenkt bzw. die Katalysatorgifte unschädlich gemacht werden. Hierzu können aus diesen Starterverbindungen mittels basischer Katalyse zunächst Vorpolymerisate hergestellt werden, welche dann nach Aufarbeitung mittels DMC-Katalyse in die erwünschten Alkylenoxidadditionsprodukte hoher Molmasse überführt werden. Unter diese Vorpolymerisate fallen bespielsweise die oben erwähnten als Starter geeigneten "vorgefertigten Alkylenoxidadditionsprodukte". Nachteilig bei dieser Vorgehensweise ist, dass solche oft mittels basischer Katalyse erhaltenen Vorpolymerisate sehr sorgfältig aufgearbeitet werden müssen, um die Deaktivierung des DMC-Katalysators durch über die Vorpolymerisate eingeschleppte basische Katalysatorspuren auszuschließen.

Dieser Nachteil kann durch das sogenannte Verfahren der kontinuierlichen Starterdosierung überwunden werden. Hierbei werden kritische Starterverbindungen im Reaktor nicht vorgelegt, sondern neben den Alkylenoxiden dem Reaktor während der Reaktion kontinuierlich zugeführt. Als Startmedium für die Reaktion können in diesem Verfahren Vorpolymerisate vorgelegt werden, auch ist die Verwendung kleiner Mengen des herzustellenden Produktes selbst als Startmedium möglich. Die Notwendigkeit, für weitere Alkylenoxidadditionen geeignete Vorpolymerisate zunächst separat herstellen zu müssen, entfällt somit.

In Variante B) der ersten Verfahrensalternative von Schritt a) des erfindungsgemäßen Verfahrens werden daher ein Starterpolyol und der DMC-Katalysator im Reaktorsystem vorgelegt und die H-funktionelle Verbindung kontinuierlich gemeinsam mit dem Alkylenoxid und dem ungesättigten cyclischen Carbonsäureanhydrid zugeführt. Als Starterpolyol in Schritt a) sind Alkylenoxidadditionsprodukte wie beispielsweise Polyetherpolyole, Polyesterpolyole, Polyetheresterpolyole, Polycarbonatpolyole, Polyestercarbonatpolyole, Polyethercarbonatpolyole jeweils beispielsweise mit OH-Zahlen im Bereich von 3 bis 1000 mg KOH/g, vorzugsweise von 3 bis 300 mg KOH/g, und / oder gemäß Schritt a) separat hergestelltes Zwischenprodukt, geeignet. Vorzugsweise wird gemäß Schritt a) separat hergestelltes Zwischenprodukt als Starterpolyol in Schritt a) eingesetzt.

In einer weniger bevorzugten Variante dieser Ausführungsform B) ist es ebenfalls möglich, das Verhältnis der Dosiergeschwindigkeiten von Alkylenoxiddosierung und der Dosierung des ungesättigten cyclischen Carbonsäureanhydrids während der Zudosierphase der drei Komponenten gegeneinander kontinuierlich oder stufenweise zu varriieren, indem beispielweise das Verhältnis des Dosierstroms des ungesättigten cyclischen Carbonsäureanhydrids zu dem des Alkylenoxids / der Epoxide Werte von 0:1 bis 1:0 annimmt. Diese Ausführungsform ist weniger bevorzugt, da nach ihr das Zwischenprodukt nach Schritt a) in weniger einheitlicher Form erhalten wird.

Vorzugsweise werden in Ausführungsform B) der ersten Verfahrensalternative von Schritt a) die Dosierung der H-funktionellen Verbindung und die des Alkylenoxids sowie des ungesättigten cyclischen Carbonsäureanhydrids gleichzeitig beendet, oder die H-funktionellen Verbindung und eine erste Teilmenge an Alkylenoxid und eine erste Teilmenge des ungesättigten cyclischen Carbonsäureanhydrids werden zunächst gemeinsam zudosiert und anschließend die zweite Teilmenge an Alkylenoxid und ungesättigtem cyclischen Carbonsäureanhydrid zudosiert, wobei die Summen der ersten und zweiten Teilmenge an Alkylenoxid und der ersten und zweiten Teilmenge an ungesättigtem cyclischen Carbonsäureanhydrid der Gesamtmenge an in Schritt a) eingesetzter Menge an einem oder mehreren Alkylenoxiden bzw. an einem oder mehreren ungesättigten cyclischen Carbonsäureanhydriden entsprechen. Die erste Teilmenge beträgt vorzugsweise 60 bis 98 Gew. -% und die zweite Teilmenge beträgt 40 bis 2 Gew.-% der insgesamt in Schritt a) zu dosierenden Menge an Alkylenoxid. Die erste Teilmenge beträgt vorzugsweise 0 bis 100 Gew. -% und die zweite Teilmenge beträgt 100 bis 0 Gew.-% der insgesamt in Schritt a) zu dosierenden Menge eines oder mehrerer ungesättigter cyclischer Carbonsäureanhydride.

Wird die Zusammensetzung der Alkylenoxide und / oder die Zusammensetzung / die Dosierrate des einen oder der mehreren ungesättigten cyclischen Carbonsäureanydride nach Ende der Dosierung der H-funktionellen Verbindung geändert, lassen sich auch nach Verfahrensvariante B) Produkte mit Multiblockstrukturen herstellen. Auch bei Verfahrensvariante B) ist es bevorzugt, dass die Dosierung des ungesättigten cyclischen Carbonsäureanhydrids vor der Alkylenoxiddosierung beendet wird, besonders bevorzugt dergestalt, dass dieser abschließende Alkylenoxidblock eine Menge von größer 1 mol Alkylenoxidpro mol aktiver H-Atome aus den als Starterverbindungen eingesetzten H-funktionellen Verbindungen umfasst. Nach Zudosierung der Reagenzien kann sich eine Nachreaktionsphase anschließen, in der der Verbrauch an Alkylenoxid / ungesättigtem cyclischen Carbonsäureanhydrid durch Überwachung des Drucks quantifiziert werden kann. Nach Erreichen von Druckkonstanz kann das Endprodukt, gegebenenfalls nach Anlegen von Vakuum oder durch Strippen zur Entfernung von nicht umgesetzen Alkylenoxiden, wie oben beschrieben, abgelassen werden.

In Variante C) der ersten Verfahrensalternative von Schritt a) des erfindungsgemäßen Verfahrens kann das doppelbindungshaltige Präpolymer vollkontinuierlich hergestellt werden. Hierzu werden neben Alkylenoxid und der H-funktionellen Verbindung sowie dem ungesättigten cyclischen Carbonsäureanhydrid auch der DMC-Katalysator dem Reaktor bzw. einem Reaktorsystem unter Alkoxylierungsbedingungen kontinuierlich zugeführt und das Produkt kontinuierlich dem Reaktor bzw. dem Reaktorsystem nach einer vorwählbaren mittleren Verweilzeit entnommen. Bei Verfahrensvariante C) ist es bevorzugt, dass als Reaktorsystem eine Reaktorkaskade eigesetzt wird, bei der sich zwischen dem Nachreaktor und dem eigentlichen Reaktor ein dritter, kontinuierlich betriebener Reaktor befindet, in den ausschließlich das eine oder mehrere Alkylenoxide kontinuierlich dosiert werden. In einer besonders bevorzugten Ausführungsform der Verfahrensvariante C) umfasst dieser abschließende Alkylenoxidblock eine Menge von größer 1 mol Alkylenoxid pro mol aktiver H-Atome aus den als Starterverbindungen eingesetzten H-funktionellen Verbindungen.

Es können sich kontinuierliche Nachreaktionsschritte, beispielsweise in einer Reaktorkaskade oder in einem Rohrreaktor anschließen. Flüchtige Bestandteile können im Vakuum und/oder durch Strippen, wie oben beschrieben, entfernt werden

Die OH-Zahlen der nach der ersten Verfahrensalternative von Schritt a) erhältlichen ungesättigten Polyetheresterpolyole weisen bevorzugt Werte von 3 mg KOH/g bis 200 mg KOH/g, besonders bevorzugt von 10 bis 60 mg KOH/g, ganz besonders bevorzugt von 20 bis 50 mg KOH/g, auf.

Die OH-Zahl kann z. B. titrimetrisch nach der Vorschrift der DIN 53240 oder spektroskopisch über NIR bestimmt werden.

Unter Äquivalentmolmasse ist die durch die Zahl der aktiven Wasserstoffatome geteilte Gesamtmasse des aktive Wasserstoffatome enthaltenden Materials zu verstehen. Im Falle von hydroxygruppenhaltigen Materialien steht sie in folgender Beziehung zur OH-Zahl:

Äquivalentmolmasse = 56100 / OH-Zahl [mg KOH/g]

Den nach Schritt a) des erfindungsgemäßen Verfahrens erhältlichen Zwischenprodukten können gegebenenfalls Alterungsschutzmittel wie z. B. Antioxidantien zugesetzt werden.

Schritt a), zweite Verfahrensalternative:

Nach einem zweiten alternativen Verfahren kann die H-funktionelle Starterverbindung zunächst mit dem ungesättigten cyclischen Carbonsäureanhydrid und anschließend mit der Alkylenoxidverbindung oder die H-funktionelle Starterverbindung gleichzeitig mit dem ungesättigten cyclischen Carbonsäureanhydrid und der Alkylenoxidverbindung umgesetzt werden. Diese Variante ist beispielsweise dann zu bevorzugen, wenn die H-funktionelle Starterverbindung eine zahlenmittlere Molmasse von 200 bis 20000 g/mol aufweist, bevorzugt von 600 bis 10000 g/mol.

Auch bei diesem Verfahren muss das unmittelbare Verfahrensprodukt vor der Weiterverwendung, beispielsweise zur Herstellung von Polyharnstoffpolyurethan- oder Polyurethanharnstoffpolymeren, nicht zwingend aufgereinigt werden. Die Verfahrensprodukte besitzen eine hohe Reinheit, insbesondere was den Anteil der unerwünschten Umesterungsprodukte betrifft und eine vergleichsweise hohe Amin- und Hydroxygruppenzahl.

Das Verfahren nach dieser zweiten Alternative kann weiterhin auch derart ausgestaltet sein, dass nach Abreaktion der vorhandenen Alkylenoxidverbindung nochmals ungesättigtes cyclisches Carbonsäureanhydrid eindosiert wird, beispielsweise etwa 1 Mol Carbonsäureanhydrid pro Mol durch die Addition der Alkylenoxidverbindung gebildeten OH-Gruppen. Es wird dann mit anderen Worten der Verfahrensschritt a) wiederholt, wobei nunmehr die ein Zerewitinoff-aktives H-Atom tragende H-funktionelle Starterverbindung das Additionsprodukt aus ursprünglicher Starterverbindung, cyclischem ungesättigtem Carbonsäureanhydrid und Alkylenoxidverbindung ist. Anschließend wird nochmals eine gewünschte Menge an Alkylenoxidverbindungen addiert, um das Hydroxylgruppen tragende Präpolymer zu erhalten. Dieses besitzt dann etwa 2 Doppelbindungen pro Hydroxylgruppe, sodass später zwei Aminfunktionalitäten über eine Michael-Addition eingeführt werden können. Die vorgenannte Reaktion lässt sich auch zwei- oder mehrfach wiederholen, sodass eine gewünschte Zahl von Aminfunktionalitäten pro ursprünglichem Zerewitinoff-aktiven H-Atom in das Hydroxy-Aminopolymer eingebaut werden können. Dies können beispielsweise 2 oder mehr, insbesondere 3 oder mehr Aminfunktionalitäten pro ursprünglichem Zerewitinoff-aktiven H-Atom sein.

Bei dieser zweiten Verfahrensalternative ist es weiter bevorzugt, dass dieses unter Einsatz eines Aminkatalysators durchgeführt wird, der vorzugsweise aus tertiären Aminen ausgewählt ist. Bei den hierbei erhaltenen Hydroxy-Aminopolymeren beträgt der Abstand zwischen der Aminfunktionalität und der Hydroxylgruppe in der Regel 6 oder 7 Bindungslängen. Der Grund hierfür liegt darin, dass bedingt durch die Aminkatalyse in der Regel nur eine Alkylenoxidverbindung mit der Carbonsäuregruppe verknüpft werden kann. Ansonsten besteht die Gefahr der Verseifung der bereits aufgebauten Esterfunktion oder die Gefahr der Umesterung. Der Aminkatalysator ist insbesondere ausgewählt aus der Gruppe umfassend:

(A1) Amine der allgemeinen Formel (2):

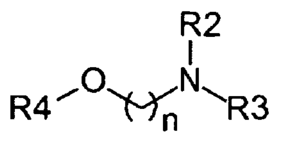

(2)

wobei gilt:

R2 und R3 sind unabhängig voneinander Wasserstoff, Alkyl oder Aryl; oder

R2 und R3 bilden gemeinsam mit dem sie tragenden N-Atom einen aliphatischen, ungesättigten oder aromatischen Heterozyklus;

n ist eine ganze Zahl von 1 bis 10;

R4 ist Wasserstoff, Alkyl oder Aryl; oder

R4 steht für $-(CH_2)_x-N(R41)(R42)$, wobei gilt:

R41 und R42 sind unabhängig voneinander Wasserstoff, Alkyl oder Aryl; oder

R41 und R42 bilden gemeinsam mit dem sie tragenden N-Atom einen aliphatischen, ungesättigten oder aromatischen Heterozyklus;

x ist eine ganze Zahl von 1 bis 10;

(B1) Amine der allgemeinen Formel (3):

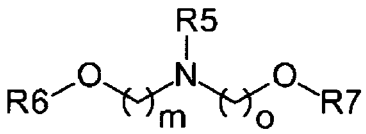

(3)

wobei gilt:

R5 ist Wasserstoff, Alkyl oder Aryl;

R6 und R7 sind unabhängig voneinander Wasserstoff, Alkyl oder Aryl;

m und o sind unabhängig voneinander eine ganze Zahl von 1 bis 10;

und/oder:

(C1) Diazabicyclo[2.2.2]octan, Diazabicyclo[5.4.0]undec-7-en, Dialkylbenzylamin, Dimethylpiperazin, 2,2'-Dimorpholinyldiethylether und/oder Pyridin.

Amine der allgemeinen Formel (2) können im weitesten Sinne als Aminoalkohole oder deren Ether beschrieben werden. Ist R4 Wasserstoff, so sind die Katalysatoren in eine Polyurethanmatrix einbaubar, wenn das erfindungsgemäße Hydroxy-Aminopolymer mit einem Polyisocyanat umgesetzt wird. Dieses ist vorteilhaft, um das Austreten des Katalysators, der im Falle von Aminen mit nachteiligen Geruchsproblemen einhergehen kann, an die Polyurethanoberfläche, die sogenannte "fogging"- oder VOC (volatile organic compounds)-Problematik, zu verhindern.

Amine der allgemeinen Formel (3) können im weitesten Sinne als Amino(bis)alkohole oder deren Ether beschrieben werden. Sind R6 und / oder R7 Wasserstoff, so sind diese Katalysatoren ebenfalls in eine Polyurethanmatrix einbaubar.

Vorzugsweise gilt hierbei, dass in dem Amin der allgemeinen Formel (2) R2 und R3 Methyl sind, R4 Wasserstoff ist und n = 2 ist oder aber R2 und R3 Methyl sind, R4 $-(CH_2)_2-N(CH_3)_2$ ist und n = 2 ist. Insgesamt ergibt sich also entweder N,N-Dimethylethanolamin oder Bis(2-(dimethylamino)ethyl)ether.

Weiterhin ist bevorzugt, dass in dem Amin der allgemeinen Formel (3) R5 Methyl ist, R6 und R7 Wasserstoff sind, m = 2 ist und o = 2 ist. Insgesamt ergibt sich also N-Methyldiethanolamin.

Besonders bevorzugte Katalysatoren sind Diazabicyclooctan, N-Methyldiethanolamin, Dimethylethanolamin, Bis(2-(dimethylamino)ethyl)ether, Diazabicyclo[5.4.0]undec-7-en, Dialkylbenzylamin, Dimethylpiperazin, 2,2'-Dimorpholinyldiethylether und Pyridin oder Kombinationen hiervon.

Die aminkatalysierte Verfahrensalternative ist zwar nicht auf den Einsatz der vorgenannten Katalysatoren beschränkt, jedoch hat sich herausgestellt, dass bestimmte Amine einen nachteiligen Einfluss auf die Reinheit des Reaktionsprodukts haben können. Dies kann sich dadurch äußern, dass es teilweise zu einer Spaltung der Esterbindungen der ungesättigten Carbonsäure, also einer Verseifung kommen kann, oder zu unerwünschten Umesterungsreaktionen. Die Nebenprodukte lassen sich zum Teil schwer entfernen beziehungsweise verschlechtern die Homogenität des Reaktionsprodukts, wenn sie nicht entfernt werden oder entfernt werden können. Aus diesem Grund sollte der Katalysator nicht Imidazol oder N-Methylimidazol sein, da diese Katalysatoren zu den vorgenannten unerwünschten Nebenreaktionen führen können. Mit anderen Worten sollten diese Verbindungen während des gesamten Reaktionsverlaufs nicht mit den Edukten oder (Zwischen)Produkten in Kontakt gebracht werden.

Was den Zugabezeitpunkt des Katalysators betrifft, so ist es von Vorteil, wenn der Katalysator gleichzeitig oder vor der Zugabe des ungesättigten cyclischen Carbonsäureanhydrids zugegeben wird.

Nach einer besonders bevorzugten Ausführungsform dieses Verfahrens wird der Aminkatalysator der Reaktionsmischung gleichzeitig oder vor der Umsetzung des Carboxylgruppen tragenden Präpolymers mit der Alkylenoxidverbindung zugesetzt. Die Menge des Katalysators, bezogen auf die Gesamtmasse des Reaktionsansatzes, kann beispielsweise ≥ 10 ppm bis ≤ 10000 ppm, bevorzugt ≥ 50 ppm bis ≤ 5000 ppm und mehr bevorzugt ≥ 100 ppm bis ≤ 2000 ppm betragen.

Im Folgenden wird Schritt b) des erfindungsgemäßen Verfahrens detailliert beschrieben. Auch diese Darstellung ist lediglich beispielhaft und nicht als die vorliegende Erfindung beschränkend zu verstehen:

Für Schritt b) wird ein geeignetes Amin bei Temperaturen von 0 °C bis 150 °C, bevorzugt 10 °C bis 100 °C und besonders bevorzugt 20 °C bis 80 °C mit den Produkten aus Schritt a) zur Reaktion gebracht. Das molare Verhältnis von primären Aminogruppen zu additionsfähigen Doppelbindungen beträgt beispielsweise etwa 1 : 1 bis 1,1 : 1. Zwar kann die Reaktion mit Kupferacetat, Zinnchlorid oder Essigsäure katalysiert werden, sie wird jedoch bevorzugt ohne Katalysatorzusatz durchgeführt.

Im Allgemeinen werden die Amine unter Inertgas dem vorgelegten Zwischenprodukt aus Schritt a) zugeführt und bei den genannten Temperaturen über einen Zeitraum von 1 h bis ca. 48 h gerührt. Eine Vorvermischung der Amine mit dem Zwischenprodukt aus Schritt a) ist ebenfalls möglich, beipielsweise über ein in der gemeinsamen Dosierstrecke befindliches Mischaggregat ("inline-blending").

Der Reaktionsfortschritt kann über gängige Methoden, wie beispielsweise online oder offline durchgeführte gaschromatographische Untersuchungen oder spektroskopische Methoden, wie beispielsweise NMR- oder IR-Spektroskopie quantifiziert werden. Spuren unreagierter Amine oder etwaige Aminüberschüsse können nach der Reaktionsphase gegebenenfalls im Vakuum bei einem absoluten Druck von 1 bis 500 mbar oder durch Strippen quantitativ entfernt werden.

Die Umsetzung einer über die erste Verfahrensalternative von Schritt a) erhaltenen Komponente mit dem oder den Aminen in Schritt b) kann prinzipiell im gleichen Reaktor wie die Herstellung der Komponente nach Schritt a) erfolgen. Es ist jedoch bevorzugt, in diesem Fall die Umsetzung nach Schritt b) in einem anderen Reaktor durchzuführen, da im Reaktor verbleibende Aminspuren die Durchführung des nächsten DMC-katalysierten Schrittes a) behindern können.

<u>Hydroxy-Aminopolymer:</u>

Wie bereits voranstehend ausgeführt wurde, betrifft die vorliegende Erfindung auch ein Hydroxy-Aminopolymer, welches nach dem erfindungsgemäßen Verfahren erhältlich ist.

In vorteilhafter Ausgestaltung des erfindungsgemäßen Hydroxy-Aminopolymers umfasst dieses Polyesterpolyoleinheiten, Polyester-Polyether-Polyoleinheiten und/oder Polyetherpolyoleinheiten, insbesondere Polyester-Polyether-Polyoleinheiten und/oder Polyetherpolyoleinheiten mit einem Anteil an Oxyethyleneinheiten von 40 bis 90 Gew.-%.

Nach einer besonders bevorzugten Ausführungsform des erfindungsgemäßen Hydroxy-Aminopolymers besitzt dieses die allgemeine Formel (I)

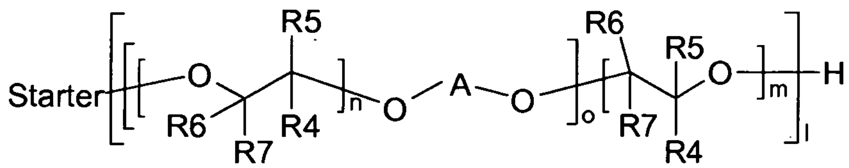

(I),

wobei

"Starter" für das Radikal der H-funktionellen Starterverbindung steht,

A für eine Aspartatgruppe folgender Struktur der Formeln (IIa) oder (IIb) steht

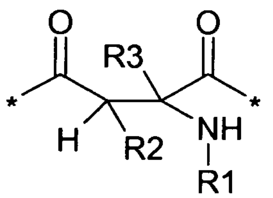

(IIa)

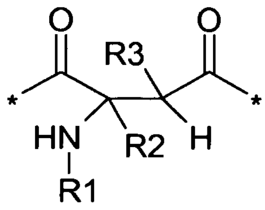

(IIb),

in denen

R1 für Wasserstoff oder einen aliphatischen, cycloaliphatischen oder aromatischen Rest steht, der auch Heteroatome, insbesondere Stickstoffatome oder Sauerstoffatome sowie Hydroxylgruppen enthalten kann,

R2 und R3 unabhängig voneinander für Wasserstoff oder einen aliphatischen oder aromatischen Rest stehen und R2 und R3 auch Bestandteil eines cycloaliphatischen Ringsystems sein können,

R4, R5, R6 und R7 unabhängig voneinander für Wasserstoff oder einen aliphatischen oder aromatischen Rest stehen und R5 und R6 auch Bestandteil eines cycloaliphatischen Ringsystems sein können,

1 der Zahl der Zerewitinoff-aktiven Wasserstoffatome der H-funktionellen Starterverbindung entspricht,

m, n und o unabhängig voneinander ganzzahlig sind, wobei n, o = 0 oder ≥ 1 und m ≥ 1 sind und n, m vorzugsweise 1 bis 430 betragen, insbesondere 2 bis 430, bevorzugt 4 bis 430, o vorzugsweise 1 bis 100, insbesondere 1 bis 50 und bevorzugt 1 bis 10 beträgt und das Verhältnis von o zu 1 im Mittel wenigstens 0,6 beträgt

und wobei die Äquivalentmolmasse der in Formel I gezeigten Struktur den Wert 18900 g/mol nicht übersteigt.

Polyharnstoffpolyurethan-System:

Ein weiterer Gegenstand der Erfindung betrifft ein Polyharnstoffpolyurethan-System, umfassend

als Komponente A) isocyanatfunktionelle Präpolymere erhältlich durch Umsetzung von

aliphatischen und/ oder aromatischen Polyisocyanaten A1) mit

Polyolen A2), die insbesondere ein zahlenmittleres Molekulargewicht von ≥ 400 g/mol und eine mittlere OH-Funktionalität von 2 bis 6 aufweisen können,

als Komponente B) ein erfindungsgemäßes Hydroxy-Aminopolymer,

gegebenenfalls als Komponente C) organische Füllstoffe, die insbesondere eine nach DIN 53019 gemessenen Viskosität bei 23 °C im Bereich von 10 bis 6000 mPas aufweisen können,
gegebenenfalls als Komponente D) Umsetzungsprodukte von isocyanatfunktionellen Präpolymeren gemäß Komponente A) mit Hydroxy-Amino-funktionellen Verbindungen gemäß Komponente B) und/oder organischen Füllstoffen gemäß Komponente C) und gegebenenfalls als Komponente E) Wasser und/oder ein tertiäres Amin.

Die erfindungsgemäßen Polyharnstoffpolyurethan-Systeme werden durch Mischung der Präpolymere A) mit der hydroxyaminofunktionellen Verbindung B) sowie gegebenenfalls den Komponenten C), D) und/oder E) erhalten. Das Verhältnis von freien oder blockierten Aminogruppen zu freien NCO-Gruppen beträgt dabei bevorzugt 1:1,5, besonders bevorzugt 1:1. Wasser und/oder Amin werden dabei der Komponente B) bzw. C) beigemischt.

Die isocyanatfunktionellen Präpolymere A) sind durch Umsetzung von Polyisocyanaten A1) mit Polyolen A2) gegebenenfalls unter Zusatz von Katalysatoren sowie Hilfs- und Zusatzstoffen erhältlich.

Als Polyisocyanate A1) können beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Polyisocyanaten A1) können auch deren höhermolekulare

Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden Polyisocyanate A1) der vorstehend genannten Art mit ausschließlich aliphatisch oder cycloaliphatisch gebundenen Isocyanatgruppen oder deren Mischungen eingesetzt.

Ebenfalls bevorzugt ist, wenn Polyisocyanate A1) der vorstehenden Art mit einer mittleren NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 verwendet werden.

Ganz besonders bevorzugt wird Hexamethylendiisocyanat als Polyisocyanat A1) eingesetzt.

Gemäß einer bevorzugten Ausführungsform des erfindungsgemäßen Polyharnstoffpolyurethan-Systems ist vorgesehen, dass die Polyole A2) Polyesterpolyole und/oder Polyester-Polyether-Polyole und/oder Polyetherpolyole sind. Insbesondere bevorzugt sind dabei Polyester-Polyether-Polyole und/oder Polyetherpolyole mit einem Ethylenoxidanteil von 40 bis 90 Gew.-%.

Bevorzugt ist auch, wenn die Polyole A2) ein zahlenmittleres Molekulargewicht von 4000 bis 8500 g/mol aufweisen.

Geeignete Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methylpentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligotetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden können. Zur ringöffnenden Polymerisation von Epoxiden können entweder Alkalihydroxide oder Doppelmetallcyanidkatalysatoren verwendet werden.

Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen können.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Ester, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil an der gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole, ausgeglichen wird.

Die Herstellung der Polyetheresterpolyole erfolgt entsprechend dem Stand der Technik bei erhöhter Temperatur im Bereich von 120 bis 250 °C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt

0,1 bis 3 mg KOH/g und besonders bevorzugt 0,15 bis 2,5 mg KOH/g absinkt.

Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di-, tri- oder höherfunktionellen Startermolekülen wie di-, tri- oder höherfunktionellen Polyolen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Ebenfalls können Hydroxylgruppen aufweisende Polycarbonate, bevorzugt Polycarbonatdiole, mit zahlenmittleren Molekulargewichten Mn von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylen-glykol, Polybutylenglykole, Bisphenol A und lactonmodifizierte Diole der vorstehend genannten Art in Frage.

Besonders bevorzugt wird zur Herstellung der Komponente A ein trifunktionelles Polyol verwendet, insbesondere ein Glycerin-gestarteter Polyether.

Zur Herstellung des Präpolymers A) kann das Polyisocyanat A1) mit dem Polyol A2) bei einem NCO/OH-Verhältnis von bevorzugt 4:1 bis 12:1, besonders bevorzugt 8:1 umgesetzt und anschließend der Anteil an nicht umgesetztem Polyisocyanat mittels geeigneter Methoden abgetrennt werden. Üblicherweise wird hierfür die Dünnschichtdestillation verwendet, wobei Präpolymere mit Restmonomergehalten von weniger als 1 Gew.-%, bevorzugt weniger als 0,1 Gew.-%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Herstellung der Präpolymere A) beträgt dabei bevorzugt 20 bis 120 °C und weiter bevorzugt 60 bis 100 °C.

Die hergestellten Präpolymere haben einen nach DIN EN ISO 11909 gemessenen mittleren NCO-Gehalt von 2 bis 10 Gew.-%, bevorzugt 2,5 bis 8 Gew.-%.

Gemäß einer weiteren Ausführungsform des erfindungsgemäßen Polyharnstoffpolyurethan-Systems können die Präpolymere A) eine mittlere NCO-Funktionalität von 1,5 bis 6, bevorzugt von 1,6 bis 4,5, weiter bevorzugt von 1,7 bis 4, ganz besonders bevorzugt von 1,8 bis 3,5 und insbesondere von 3 aufweisen.

Bei den organischen Füllstoffen der Komponente C) kann es sich bevorzugt um hydroxyfunktionelle Verbindungen handeln, insbesondere um Polyetherpolyole mit sich wiederholenden Ethylenoxid Einheiten.

Vorteilhaft ist auch, wenn die Füllstoffe der Komponente C) eine mittlere OH-Funktionalität von 1,5 bis 3, bevorzugt von 1,8 bis 2,2 und besonders bevorzugt von 2 aufweisen.

Beispielsweise können als organische Füllstoffe bei 23 °C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Die Viskosität der organischen Füllstoffe, gemessen nach DIN 53019 bei 23 °C, beträgt bevorzugt 50 bis 4000 mPas, besonders bevorzugt 50 bis 2000 mPas.

In einer bevorzugten Ausführungsform des erfindungsgemäßen Polyharnstoffpolyurethan-Systems werden als organische Füllstoffe Polyethylenglykole eingesetzt. Diese haben bevorzugt ein zahlenmittleres Molekulargewicht von 100 bis 1000 g/mol, besonders bevorzugt 200 bis 400 g/mol.

Um das mittlere Äquivalentgewicht der insgesamt zur Präpolymervernetzung eingesetzten Verbindungen bezogen auf die NCO-reaktiven Gruppen weiter zu reduzieren, ist es möglich zusätzlich Umsetzungsprodukte der Präpolymere A) mit der aminofunktionellen Verbindung B) und/oder den organischen Füllstoffen C), sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1, besonders bevorzugt 15 zu 1 bis 4 zu 1 eingestellt.

Vorteil dieser Modifizierung durch Vorverlängerung ist, dass das Äquivalentgewicht und das Äquivalentvolumen der Härterkomponente in größeren Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden kann.

Gemäß einer weiteren bevorzugten Ausführungsform des erfindungsgemäßen Polyharnstoffpolyurethan-Systems ist vorgesehen, dass die Komponente E) ein tertiäres Amin der allgemeinen Formel (IX)

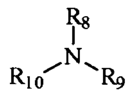
(IX)

enthält, in der

$R_8$, $R_9$, $R_{10}$ unabhängig voneinander Alkyl- oder Heteroalkylreste mit Heteroatomen in der Alkylkette oder an deren Ende sein können, oder $R_8$ und $R_9$ gemeinsam mit dem sie tragenden Stickstoffatom einen aliphatischen, ungesättigten oder aromatischen Heterozyklus bilden können, der gegebenenfalls weitere Heteroatome enthalten kann.

Diese Polyharnstoffpolyurethan-Systeme zeichnen sich durch eine besonders schnelle Aushärtung aus.

Bei den in Komponente E) verwendeten Verbindungen kann es sich ganz besonders bevorzugt um tertiäre Amine ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol, 3,3',3"-(1,3,5-Triazinan-1,3,5-triyl)tris(N,N-dimethyl-propan-1-amin) handeln.

Ganz besonders hohe Aushärtungsgeschwindigkeiten können auch erzielt werden, wenn die Komponente E) 0,2 bis 2,0 Gew.-% Wasser und/oder 0,1 bis 1,0 Gew.-% des tertiäres Amin enthält.

Nach einer besonders bevorzugten Ausführungsform des erfindungsgemäßen Polyharnstoffpolyurethan-Systems entspricht das Umsetzungsprodukt der Komponenten A1 und A2 einem trifunktionellen Isocyanat der allgemeinen Formel (X)

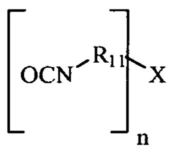
(X),

insbesondere der allgemeinen Formel (XI),

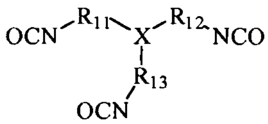
(XI)

entspricht, wobei X in Formel (X) für ein n- beziehungsweise bei Formel (XI) für ein dreiwertiges organisches Radikal steht, wie beispielsweise ein Glycerinradikal, und $R_{11}$, $R_{12}$ und $R_{13}$ unabhängig voneinander gleiche oder verschiedene organische Reste ohne Zerewitinoff-aktives H-Atom darstellen. Bevorzugt enthalten die Reste $R_{11}$, $R_{12}$ und $R_{13}$ Oxyalkyleneinheiten oder bestehen daraus. Hierbei ist es weiter bevorzugt, wenn die Oxyalkyleneinheiten einen Anteil an Oxyethyleneinheiten von 40 bis 90 Gew.-% aufweisen. Die restlichen Oxyalkyleneinheiten werden insbesondere durch Oxypropyleneinheiten gebildet.

Selbstverständlich können in die Polyharnstoffpolyurethan-Systeme auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe eingearbeitet werden.

Die Wirkstoffe können als reiner Wirkstoff oder aber in verkapselter Form vorliegen, um beispielsweise eine zeitlich verzögerte Abgabe zu erzielen. Als medizinische Wirkstoffe können im Rahmen der vorliegenden Erfindung eine Vielzahl von Wirkstofftypen und -klassen eingesetzt werden.

Ein solcher medizinischer Wirkstoff kann beispielsweise eine unter in vivo-Bedingungen Stickstoffmonoxidfreisetzende Komponente, bevorzugt L-Arginin oder eine L-Argininhaltige oder eine L-Arginin freisetzende Komponente, besonders bevorzugt L-Arginin Hydrochlorid umfassen. Auch Prolin, Ornithin und/oder andere biogene Zwischenstufen wie beispielsweise biogene Polyamine (Spermin, Spermitin, Putrescin oder bioaktive künstliche Polyamine) können

verwendet werden. Derartige Komponenten unterstützen bekanntermaßen die Wundheilung, wobei deren kontinuierliche mengenmäßig nahezu gleichmäßige Abgabe der Wundheilung besonders zuträglich ist.

Weitere erfindungsgemäß verwendbare Wirkstoffe umfassen mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, der pflanzlichen wundheilungsfördernden Substanzen oder Substanzgemischen, Pflanzenextrakte, Enzyme, Wachstumsfaktoren, Enzyminhibitoren sowie Kombinationen hiervon.

Als Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indometacin®, Ketoprofen®, Piroxicam® geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF $\alpha$; (Transforming Growth Factor alpha), TGF ß (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere ß-Carotin, Vitamin E, Gruppe der Tocopherole, insbesondere $\alpha$ Tocopherol, ß-Tocopherol, $\gamma$-Tocopherol, $\delta$-Tocopherol und $\alpha$-Tocotrienol, ß-Tocotrienol, $\gamma$-Tocotrienol und $\delta$-Tocotrienol, Vitamin K, Phyllochinon insbesondere Phytomenadion oder pflanzliches Vitamin K, Vitamin C, L-Ascorbinsäure, Vitamin B 1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das gemizid, bakterizid, bakteriostatisch, fungizid, viruzid, virustatisch und/ oder allgemein mikrobiozid wirkt.

Insbesondere sind solche Stoffe geeignet, die ausgewählt werden aus der Gruppe Resorcinol, Iod, Iod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobiellen Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche, wundheilungsfördernde Wirkstoffe sind im Zusammenhang mit der vorliegenden Erfindung insbesondere Extrakte der Kamille, Hamamelis-Extrakte z.B. Hamamelis virgina, Calendula-Extrakt, Aloe- Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu nennen.

Der Gehalt der Wirkstoffe richtet sich dabei in erster Linie an der medizinisch erforderlichen Dosis aus sowie auch an der Verträglichkeit mit den übrigen Bestandteilen der erfindungsgemäßen Zusammensetzung.

Das erfindungsgemäße Polyharnstoffpolyurethan-System ist besonders zum Verschließen, Verbinden, Verkleben oder Abdecken von Zellgewebe und insbesondere zur Stillung des Austritts von Blut oder Gewebeflüssigkeiten oder dem Verschluss von Leckagen in Zellgewebe geeignet. Ganz besonders bevorzugt kann es zur Verwendung oder zur Herstellung eines Mittels zum Verschließen, Verbinden, Verkleben oder Abdecken von menschlichem oder tierischem Zellgewebe verwendet werden. Mit seiner Hilfe können schnell aushärtende, stark am Gewebe anhaftende, transparente, flexible und biokompatible Klebnähte hergestellt werden.

Noch ein weiterer Gegenstand der Erfindung ist ein Dosiersystem mit zwei Kammern für ein erfindungsgemäßes Polyharnstoffpolyurethan-System, bei dem in der einen Kammer die Komponente A) und in der anderen Kammer die Komponenten B) und gegebenenfalls die Komponenten C), D) und E) des Polyharnstoffpolyurethan-Systems enthalten sind. Ein derartiges Dosiersystem eignet sich insbesondere dafür, das Polyharnstoffpolyurethan-System als Kleber auf Gewebe zu applizieren.

Die vorliegende Erfindung wird im Folgenden anhand von Ausführungsbeispielen näher erörtert.

Mess- und Bestimmungsmethoden:

Molmassen: Die Molmassen wurden mittels Gelpermeationschromatographie (GPC) wie folgt bestimmt: Die Kalibrierung erfolgte mit Polystyrol-Standards mit Molmassen von Mp 1.000.000 bis 162. Als Eluent wurde Tetrahydrofuran p.A. verwendet. Die folgenden Parameter wurden bei der Doppelmessung eingehalten: Entgasung: Online - Degasser; Durchfluß: 1 ml/Min; Analysenzeit: 45 Minuten; Detektoren: Refraktometer und UV-Detektor; Injektionsvolumen: 100 $\mu$l - 200 $\mu$l. Die Berechnung der Molmassenmittelwerte Mw, Mn und Mp sowie der Polydispersität Mw/Mn erfolgte softwaregestützt. Basislinienpunkte und Auswertegrenzen wurden entsprechend der DIN 55672 Teil 1 festgelegt.

Die Bestimmung der OH-Zahlen erfolgte gemäß der Vorschrift der DIN 53240.

Die Bestimmung der Säure-Zahlen erfolgte gemäß der Vorschrift der DIN EN ISO 2114.

| | |
|---|---|
| NCO-Gehalt: | Wenn nicht ausdrücklich anders erwähnt, volumetrisch gemäß DIN-EN ISO 11909 bestimmt. |
| Viskosität: | Wurde nach ISO 3219 bei 23°C bestimmt. |
| Restmonomergehalt: | Bestimmt nach DIN ISO 17025 |

Substanzen:

HDI: Hexamethylendiisocyanat (Bayer MaterialScience AG)

Soweit nicht anders angegeben wurden die eingesetzten Chemikalien von Aldrich oder Acros bezogen.

Herstellung der Hydroxy-Aminopolymere

Beispiel 1: Herstellung eines trifunktionellen Hydroxy-Aminopolymers:

In einem 1 l Laborautoklaven wurden unter Stickstoffatmosphäre 650 g (0,146 mol) eines trifunktionellen, Glycerin-gestarteten Polyetherpolyols mit einem Ethylenoxid/Propylenoxid-Verhältnis von 73/27 (w/w) und OH-Zahl = 37,9 mg KOH/g (Molmasse 4440 g/mol) vorgelegt und dann auf 60°C aufgeheizt. Bei dieser Temperatur wurden 41,8 g (0,426 mol) Maleinsäureanhydrid und 0,73 g N-Methyldiethanolamin zugegeben und anschließend 60 Minuten bei 60°C gerührt. Danach wurde auf 90°C aufgeheizt, bei dieser Temperatur innerhalb von 30 Minuten 77,4 g (1,756 mol) Ethylenoxid in den Autoklaven dosiert und anschließend 5 h bei dieser Temperatur nachreagiert. Leichtflüchtige Bestandteile wurden bei 90°C für 60 Minuten im Vakuum ausgeheizt und der Reaktionsansatz dann auf Raumtemperatur abgekühlt.

Man erhielt ein Vorprodukt mit einer OH-Zahl von 35,5 mg KOH/g und einer Säure-Zahl von 0,12 mg KOH/g.

Michael-Addition von Pentylamin:

Zu 11,85 g des Vorprodukts wurden 0,65 g (3 Equivalente) Pentylamin zugesetzt. Die Reaktionsmischung wurde 6 h bei 60°C im Heizblock gerührt. Anschließend wurde eventuell überschüssiges Amin im Hochvakuum entfernt.

Analog wurden die nachfolgenden Amine umgesetzt:

Tabelle 1

| Amin für Michael Addition | Einwaage Amin | Hydroxy-Aminopolymer |
|---|---|---|
| N-Pentylamin | 0,63 g | 2 |
| N-Propylamin | 0,44 g | 3 |
| N-Butylamin | 0,54 g | 4 |
| N-Hexylamin | 0,76 g | 5 |
| N-Decylamin | 1,16 g | 6 |
| 3-Methoxypropan-1-amin | 0,66 g | 7 |
| Cyclopentylamin | 0,63 g | 8 |
| 2-(Morpholin-4-yl)ethanamin | 0,96 g | 9 |

Bei den in Tabelle 1 aufgeführten Verbindungen handelt es sich also um erfindungsgemäße Hydroxy-Aminopolymere. Diese werden im Folgenden mit einem trifunktionellen NCO-terminierten Präpolymer umgesetzt. Die Herstellung des trifunktionellen NCO-terminierten Präpolymers gestaltet sich wie folgt:

Herstellung eines trifunktionellen NCO-terminierten Präpolymers

465 g HDI und 2.35 g Benzoylchlorid wurden in einem 1 l Vierhalskolben vorgelegt. Innerhalb von 2 h wurden

bei 80°C 931.8 g eines trifunktionellen Polyethers der Molmasse 4500, gestartet auf Glycerin und einem Ethylenoxidgehalt von 71 % und einem Propylenoxidgehalt von 29 %, jeweils bezogen auf dem gesamten Alkylenoxidgehalt, hinzugefügt und 1 h nachgerührt. Anschließend wurde durch Dünnschichtdestillation bei 130 °C und 0,13 mbar das überschüssige HDI abdestilliert. Man erhielt 980 g (71 %) des Präpolymers mit einem NCO-Gehalt von 2,37 % und einer Viskosität von 4500 mPas/23°C. Der Restmonomerengehalt betrug < 0,03 % HDI.

Gewebekleber

Zu 2 g des trifunktionellen NCO-terminierten Präpolymers wurden 2.08 g (1 Equivalent) des Hydroxy-Aminopolymers 2 zugesetzt und in einem Becher 20 s gut verrührt. Das Polyharnstoffpolyurethan-System wurde unmittelbar danach auf das zu klebende Muskelgewebe als dünne Schicht aufgetragen. Als Verarbeitungszeit wurde dabei die Zeit bestimmt, innerhalb der das Klebstoff-System noch eine so niedrige Viskosität besaß, dass es problemlos auf das Gewebe aufgetragen werden konnte.

Die Zeit, nach der das Polyharnstoffpolyurethan-System nicht mehr klebrig war (tack free time) wurde durch Haftversuche mit einem Glasstab gemessen. Dazu wurde der Glasstab mit der Schicht aus dem Polyharnstoffpolyurethan-System in Kontakt gebracht. Blieb dieser nicht mehr haften, wurde die System als tack free angesehen. Zusätzlich wurde die Klebekraft bestimmt, indem zwei Stücke Muskelgewebe (1 = 4 cm, h = 0.3 cm, b = 1 cm) an den Enden 1 cm weit mit dem Polyharnstoffpolyurethan-System bestrichen und überlappend geklebt wurden. Die Klebkraft des Polyharnstoffpolyurethan-Systems wurde jeweils durch Zug überprüft.

Umsetzung des NCO-terminierten Präpolymers mit den Hydroxy-Aminopolymeren aus Tabelle 1:

Tabelle 2

| Hydroxy-Aminopolymer | Produktnummer Aushärtung | Verarbeitungszeit | Tack free time | Klebekraft |
|---|---|---|---|---|
| N-Pentylamin/ (2) | 2a | 1 min 30 s | 3 min | ++ |
| N-Propylamin/ (3) | 3a | 1 min 20 s | 3 min | + |
| N-Butylamin/ (4) | 4a | 1 min 30 s | 3 min | ++ |
| N-Hexylamin/ (5) | 5a | 1 min 10 s | 3 min 30 s | ++ |
| N-Decylamin/ (6) | 6a | 1 min 20 s | 3 min 30 s | ++ |
| 3-Methoxypropan-1-amin/ (7) | 7a | 1 min 10 s | 4 min | + |
| Cyclopentylamin/ (8) | 8a | 1 min 40 s | 6 min | 0 |
| 2-(Morpholin-4-yl)ethanamin/ (9) | 9a | 1 min | 7 min | + |

Die Exothermie der Aushärtung lag bei allen Beispielen zwischen 23 und 25°C.

Bestimmung der Bioabbaubarkeit

Der Klebstoff wurde in einem Rohr (Durchmesser 0,5 cm, Länge 2 cm) zur Aushärtung gebracht. Der dabei entstandene 2.7 g schwere Prüfkörper wurde in 10 ml Pufferlösung (pH 7,4, Aldrich P-5368) so lange bei 60°C bzw. 37°C im Schüttelinkubator mit 150 U/min geschüttelt, bis sich das Material vollständig, d.h. ohne Bodensatz aufgelöst hatte.

Alle Proben waren nach 4 Tagen bei 60°C vollständig abgebaut.

Cytotoxizitätsmessung von 2a

Der ausgehärtete Klebstoff wurde gemäß ISO 10993-5:2009 mit L 929 Zellen auf Zytotoxizität geprüft. Das Material hat sich als nicht zytotoxisch erwiesen.

## Patentansprüche

1. Verfahren zur Herstellung eines Hydroxy-Aminopolymers umfassend die Schritte:

   a) Umsetzen einer wenigstens ein Zerewitinoff-aktives H-Atom tragenden H-funktionellen Starterverbindung

mit einem ungesättigten cyclischen Carbonsäureanhydrid und wenigstens einer Alkylenoxidverbindung zum Erhalt eines Hydroxylgruppen tragenden Präpolymers,

b) Addition eines primären Amins und/oder von Ammoniak an die Doppelbindung(en) des nach Schritt a) erhaltenen Hydroxylgruppen tragenden Präpolymers zum Erhalt des Hydroxy-Aminopolymers,

wobei das Verhältnis der addierten Aminogruppen zu den Hydroxylgruppen im Hydroxy-Aminopolymer wenigstens 0,6 beträgt.

2. Verfahren nach Anspruch 1, **dadurch gekennzeichnet, dass**

• das Verhältnis der addierten Aminogruppen zu den Hydroxylgruppen im Hydroxy-Aminopolymer 0,8 bis 2,5 beträgt, und/ oder dass das Hydroxy-Aminopolymer eine OH-Funktionalität von 1,5 bis 6 aufweist, und/ oder
• dass die H-funktionelle Starterverbindung 1 bis 35 Zerewitinoff-aktive H-Atome aufweist.

3. Verfahren nach Anspruch 1 oder 2, **dadurch gekennzeichnet, dass** das ungesättigte cyclische Carbonsäureanhydrid ausgewählt ist aus ungesättigten cyclischen Dicarbonsäureanhydriden, wie Maleinsäureanhydrid, Tetrahydrophthalsäureanhydrid, insbesondere 3,4,5,6-Tetrahydrophthalsäureanhydrid, sowie Kombinationen hiervon.

4. Verfahren nach einem der vorstehenden Ansprüche, **dadurch gekennzeichnet, dass** die Alkylenoxidverbindung ausgewählt ist aus solchen mit 2 bis 24 Kohlenstoffatomen.

5. Verfahren nach einem der vorstehenden Ansprüche, **dadurch gekennzeichnet, dass** das Stoffmengenverhältnis zwischen der Alkylenoxidverbindung und dem Carbonsäureanhydrid wenigstens 1 : 1, beträgt.

6. Verfahren nach einem der vorstehenden Ansprüche, **dadurch gekennzeichnet, dass** die H-funktionelle Starterverbindung zunächst mit einer ersten Menge der Alkylenoxidverbindung und anschließend mit dem ungesättigten cyclischen Carbonsäureanhydrid und einer weiteren Menge der Alkylenoxidverbindung umgesetzt wird, wobei die H-funktionelle Starterverbindung vorzugsweise eine zahlenmittlere Molmasse von 17 bis 1200 g/mol aufweist.

7. Verfahren nach einem der vorstehenden Ansprüche, **dadurch gekennzeichnet, dass** die Umsetzung der H-funktionellen Starterverbindung mit dem ungesättigten cyclischen Carbonsäureanhydrid und/ oder die Addition der Alkylenoxidverbindung unter Einsatz eines Doppelmetallcyanid-Katalysators (DMC-Katalysator) durchgeführt wird.

8. Verfahren nach einem der Ansprüche 1 bis 5, **dadurch gekennzeichnet, dass** die H-funktionelle Starterverbindung zunächst mit dem ungesättigten cyclischen Carbonsäureanhydrid und anschließend mit der Alkylenoxidverbindung umgesetzt wird oder dass die H-funktionelle Starterverbindung gleichzeitig mit dem ungesättigten cyclischen Carbonsäureanhydrid und der Alkylenoxidverbindung umgesetzt wird, und/oder wobei das Verfahren unter Einsatz eines Aminkatalysators durchgeführt wird.

9. Hydroxy-Aminopolymer erhältlich nach einem Verfahren gemäß einem der Ansprüche 1 bis 8, wobei das Hydroxy-Aminopolymer insbesondere Polyesterpolyoleinheiten, Polyester-Polyether-Polyoleinheiten und/oder Polyetherpolyoleinheiten umfasst, vorzugsweise Polyester-Polyether-Polyoleinheiten und/oder Polyetherpolyoleinheiten mit einem Anteil an Oxyethyleneinheiten von 40 bis 90 Gew.-%, wobei das Hydroxy-Aminopolymer bevorzugt die allgemeine Formel (I) besitzt

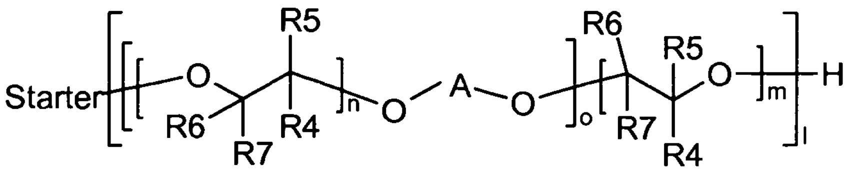

(I),

wobei

"Starter" für das Radikal der H-funktionellen Starterverbindung steht,
A für eine Aspartatgruppe folgender Struktur der Formeln (IIa) oder (IIb) steht

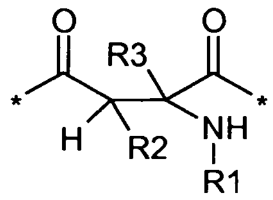
(IIa)

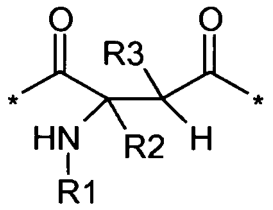
(IIb),

in denen

R1 für Wasserstoff oder einen aliphatischen, cycloaliphatischen oder aromatischen Rest steht, der auch Heteroatome, insbesondere Stickstoffatome oder Sauerstoffatome sowie Hydroxylgruppen enthalten kann,
R2 und R3 unabhängig voneinander für Wasserstoff oder einen aliphatischen oder aromatischen Rest stehen und R2 und R3 auch Bestandteil eines cycloaliphatischen Ringsystems sein können,
R4, R5, R6 und R7 unabhängig voneinander für Wasserstoff oder einen aliphatischen oder aromatischen Rest stehen und R5 und R6 auch Bestandteil eines cycloaliphatischen Ringsystems sein können,
1 der Zahl der Zerewitinoff-aktiven Wasserstoffatome der H-funktionellen Starterverbindung entspricht,
m, n und o unabhängig voneinander ganzzahlig sind, wobei n, o = 0 oder $\geq$ 1 und m $\geq$ 1 sind und das Verhältnis von o zu 1 im Mittel wenigstens 0,6 beträgt

und wobei die Äquivalentmolmasse der in Formel I gezeigten Struktur den Wert 18900 g/mol nicht übersteigt.

**10.** Polyharnstoffpolyurethan-System, umfassend

als Komponente A) isocyanatfunktionelle Präpolymere erhältlich durch Umsetzung von

aliphatischen und/ oder aromatischen Polyisocyanaten A1) mit
Polyolen A2),

als Komponente B) ein Hydroxy-Aminopolymer gemäß Anspruch 9,

gegebenenfalls als Komponente C) organische Füllstoffe,
gegebenenfalls als Komponente D) Umsetzungsprodukte von isocyanatfunktionellen Präpolymeren gemäß Komponente A) mit Hydroxy-Amino-funktionellen Verbindungen gemäß Komponente B) und/oder organischen Füllstoffen gemäß Komponente C) und gegebenenfalls als Komponente E) Wasser und/oder ein tertiäres Amin

**11.** Polyharnstoffpolyurethan-System nach Anspruch 10, **dadurch gekennzeichnet, dass** zur Herstellung der Komponente A ein trifunktionelles Polyol verwendet wird,

**12.** Polyharnstoffpolyurethan-System nach Anspruch 10 oder 11, **dadurch gekennzeichnet, dass** die Komponente E) ein tertiäres Amin der allgemeinen Formel (IX)

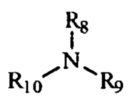
(IX)

enthält, in der $R_8$,$R_9$ und $R_{10}$unabhängig voneinander Alkyl- oder Heteroalkyreste mit Heteroatomen in der Alkylkette

oder an deren Ende sein können, oder $R_8$ und $R_9$ gemeinsam mit dem sie tragenden Stickstoffatom einen aliphatischen, ungesättigten oder aromatischen Heterozyklus bilden können, der gegebenenfalls weitere Heteroatome enthalten kann.

**13.** Polyharnstoffpolyurethan-System nach einem der Ansprüche 10 bis 12, **dadurch gekennzeichnet, dass** das tertiäre Amin ausgewählt ist aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol, 3,3',3"-(1,3,5-Triazinan-1,3,5-triyl)tris(N,N-dimethyl-propan-1-amin).

**14.** Polyharnstoffpolyurethan-System nach einem der Ansprüche 10 bis 13 zum Verschließen, Verbinden, Verkleben oder Abdecken von Zellgewebe,
oder dem Verschluss von Leckagen in Zellgewebe.

**15.** Dosiersystem mit zwei Kammern für ein Polyharnstoffpolyurethan-System nach einem der Ansprüche 10 bis 13, **dadurch gekennzeichnet, dass** in der einen Kammer die Komponente A) und in der anderen Kammer die Komponenten B) und gegebenenfalls die Komponenten C), D) und E) des Polyharnstoffpolyurethan-Systems enthalten sind.

## Claims

**1.** Process for producing a hydroxy amino polymer comprising the steps:

a) Reaction of an H functional starter compound bearing at least one Zerewitinoff active H atom with an unsaturated, cyclical carboxylic acid anhydride and at least one alkylene oxide compound for obtaining a prepolymer bearing hydroxyl groups,
b) Addition of a primary amine and/or of ammonia to the double bond(s) of the prepolymer bearing hydroxyl groups obtained according to step a) for obtaining the hydroxy amino polymer,

wherein the ratio of added amino groups to hydroxyl groups in a hydroxy amino polymer is at least 0.6.

**2.** Process according to Claim 1, wherein

• the ratio of added amino groups to hydroxyl groups in said hydroxy amino polymer is 0.8 to 2.5 and/or wherein said hydroxy amino polymer has an OH functionality of 1.5 to 6 and/or
• wherein said H functional starter compound has 1 to 35 Zerewitinoff active H atoms.

**3.** Process according to Claim 1 or 2, wherein said unsaturated, cyclical carboxylic acid anhydride is selected from unsaturated, cyclical dicarboxylic acid anhydrides, such as maleic acid anhydride, tetrahydrophthalic acid anhydride, particularly 3,4,5,6-Tetrahydrophthalic acid anhydride, as well as combinations thereof.

**4.** Process according to one of the previous Claims, wherein said alkylene oxide compound is selected from those with 2 to 24 carbon atoms.

**5.** Process according to one of the previous Claims, wherein a molar ratio between said alkylene oxide compound and carboxylic acid anhydride is at least 1 : 1.

**6.** Process according to one of the previous Claims, wherein said H functional starter compound is first reacted with an initial amount of alkylene oxide compound and then with the unsaturated, cyclical carboxylic acid anhydride and an additional amount of alkylene oxide compound, wherein said H functional starter compound preferably has a number average molar mass of 17 to 1200 g/mol.

**7.** Process according to one of the previous Claims, wherein the reaction of said H functional starter compound with said unsaturated, cyclical carboxylic acid anhydride and/or the addition of said alkylene oxide compound is performed using a double metal cyanide catalyst (DMC catalyst).

**8.** Process according to one of the Claims 1 to 5, wherein said H functional starter compound is first reacted with said unsaturated, cyclical carboxylic acid anhydride and subsequently with said alkylene oxide compound or wherein

said H functional starter compound is simultaneously reacted with said unsaturated, cyclical carboxylic acid anhydride and said alkylene oxide compound, and/or wherein the process is conducted using an amine catalyst.

**9.** Hydroxy amino polymer available pursuant to a process according to one of the Claims 1 to 8, wherein said hydroxy amino polymer comprises in particular polyester polyol units, polyester-polyether polyol units, and/or polyether polyol units, preferably polyester-polyether polyol units, and/or polyether polyol units having a share of oxyethylene units of 40 to 90% by weight, wherein said hydroxy amino polymer preferably has a general formula (I)

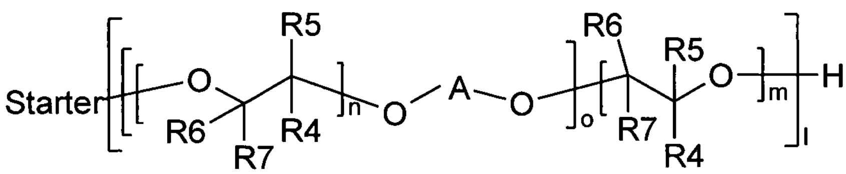

(I),

wherein

"starter" represents the radical of the H functional starter compound,
A represents an aspartate group of the following structure of formulas (IIa) or (IIb)

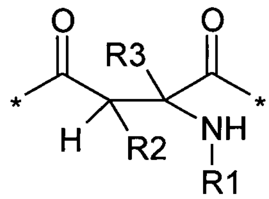

(IIa)

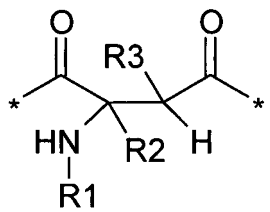

(IIb),

in which

R1 represent hydrogen or an aliphatic, cycloaliphatic or aromatic radical, which may also contain heteroatoms, particularly nitrogen atoms or oxygen atoms as well as hydroxyl groups,
R2 and R3 independently represent hydrogen or an aliphatic or aromatic radical and R2 and R3 may also be a component of a cycloaliphatic ring system,
R4, R5, R6, and R7 independently represent hydrogen or an aliphatic or aromatic radical and R5 and R6 may also be a component of a cycloaliphatic ring system,
I corresponds to the number of Zerewitinoff active hydrogen atoms of the H functional starter compound,
m, n, and o are independently whole numbers, wherein n, o = 0 or ≥ 1 and m ≥ 1 and the ratio of o to 1 in the medium is at least 0.6

and wherein the equivalent molar mass of a structure shown in formula I does not exceed the value of 18900 g/mol.

**10.** Polyurea-polyurethane system comprising
isocyanate functional prepolymers as a component A) can be achieved through a reaction of

aliphatic and / or aromatic polyisocyanates A1) with
polyols A2),

a hydroxy amino polymer according to Claim 9 as component B),
potentially organic fillers as a component C),
reaction products of isocyanate functional prepolymers according to component A) with hydroxy amino functional compounds according to component B), and/or organic fullers according to component C) potentially as component D), and
potentially water and/or a tertiary amine as a component E).

**11.** Polyurea-polyurethane system according to Claim 10, wherein a trifunctional polyol is used for producing component A.

**12.** Polyurea-polyurethane system according to Claim 10 or 11, wherein a component E) contains a tertiary amine of a general formula (IX),

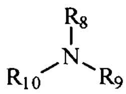 (IX)

in which $R_8$,$R_9$, and $R_{10}$ may independently be alkyl or heteroalkyl radicals having heteroatoms in an alkyl chain or at their ends, or $R_8$ and $R_9$ can form an aliphatic, unsaturated or aromatic heterocycle together with a nitrogen atom bearing them, which may potentially contain additional heteroatoms.

**13.** Polyurea-polyurethane system according to one of the Claims 10 to 12, wherein said tertiary amine is selected from a group of triethanolamine, tetrakis (2-hydroxyethyl) ethylenediamine, N,N-dimethyl-2-(4-methylpiperazine-1-yl)ethanamine, 2-{[2-(dimethylamino)ethyl](methyl) amino} ethanol, 3,3',3"-(1,3,5-triazinane-1,3,5-triyl)tris(N,N-dimethyl-propane-1-amine).

**14.** Polyurea-polyurethane system according to one of the Claims 10 to 13 for closing, bonding, adhering or covering cell tissue or for closing leakages in cell tissue.

**15.** Dispensing system having two chambers for a polyurea-polyurethane system according to one of the Claims 10 to 13, wherein said component A) is contained in one chamber and said component B) and potentially said components C), D), and E) of said polyurea-polyurethane system in another chamber.

## Revendications

**1.** Procédé de fabrication d'un hydroxyaminopolymère comprenant les étapes de :

a) mise en réaction d'un composé de départ H-fonctionnel portant au moins un atome d'hydrogène actif de Zerewitinoff avec un anhydride d'acide carboxylique cyclique insaturé et au moins un composé d'oxyde d'alkylène pour obtenir un prépolymère porteur de groupes hydroxyle,
b) addition d'une amine primaire et/ou d'ammoniac à la/aux double(s) liaison(s) du prépolymère porteur de groupes hydroxyle obtenu suivant l'étape a) pour obtenir l'hydroxyaminopolymère,

où le rapport des groupes amino ajoutés aux groupes hydroxyle dans l'hydroxyaminopolymère s'élève à au moins 0,6.

**2.** Procédé selon la revendication 1, **caractérisé en ce que**

• le rapport des groupes amino ajoutés aux groupes hydroxyle dans l'hydroxyaminopolymère est 0,8 à 2,5, et/ou l'hydroxyaminopolymère présente une fonctionnalité OH de 1,5 à 6,
et/ou
• le composé de départ H-fonctionnel présente 1 à 35 atomes d'hydrogène actifs de Zerewitinoff.

**3.** Procédé selon la revendication 1 ou 2, **caractérisé en ce que** l'anhydride d'acide carboxylique cyclique insaturé est sélectionné parmi des anhydrides d'acide dicarboxylique cycliques insaturés, tels que l'anhydride d'acide maléique, l'anhydride d'acide tétrahydrophtalique, en particulier l'anhydride de 3,4,5,6-acide tétrahydrophtalique, ainsi que des combinaisons de ceux-ci.

4. Procédé selon l'une des revendications précédentes, **caractérisé en ce que** le composé d'oxyde d'alkylène est sélectionné parmi ceux ayant 2 à 24 atomes de carbone.

5. Procédé selon l'une des revendications précédentes, **caractérisé en ce que** le rapport molaire entre le composé d'oxyde d'alkylène et l'anhydride d'acide carboxylique est au moins 1:1.

6. Procédé selon l'une des revendications précédentes, **caractérisé en ce que** le composé de départ H-fonctionnel est mis en réaction, dans un premier temps, avec une première quantité du composé d'oxyde d'alkylène, puis avec l'anhydride d'acide carboxylique cyclique insaturé et une quantité supplémentaire du composé d'oxyde d'alkylène, le composé de départ H-fonctionnel présentant de préférence un poids moléculaire moyen en nombre de 17 à 1200 g/mol.

7. Procédé selon l'une des revendications précédentes, **caractérisé en ce que** la mise en réaction du composé de départ H-fonctionnel avec l'anhydride d'acide carboxylique cyclique insaturé et/ou l'addition du composé d'oxyde d'alkylène est effectuée en présence d'un catalyseur de cyanure métallique double (CMD).

8. Procédé selon l'une des revendications 1 à 5, **caractérisé en ce que** le composé de départ H-fonctionnel est mis en réaction, dans un premier temps, avec l'anhydride d'acide carboxylique cyclique insaturé, puis avec le composé d'oxyde d'alkylène, ou que le composé de départ H-fonctionnel est mis en réaction simultanément avec l'anhydride d'acide carboxylique cyclique insaturé et le composé d'oxyde d'alkylène, et/ou le procédé étant réalisé en présence d'un catalyseur aminé.

9. Hydroxyaminopolymère pouvant être obtenu suivant un procédé selon l'une des revendications 1 à 8, l'hydroxyaminopolymère comprenant en particulier des unités polyesterpolyol, des unités polyester-polyéther-polyol et/ou des unités polyétherpolyol, de préférence des unités polyester-polyéther-polyol et/ou des unités polyétherpolyol ayant une part en unités oxyéthylène comprise entre 40 et 90 % en poids, l'hydroxyaminopolymère possédant de préférence la formule générale (I)

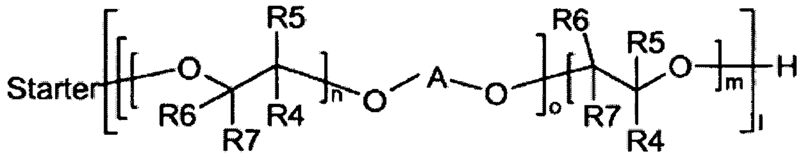

(I),

où

« Starter » (amorceur) représente le radical du composé de départ H-fonctionnel,
A représente un groupe aspartate de structure suivante, de formule (IIa) ou (IIb),

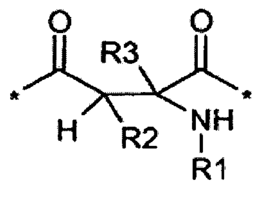

(IIa)

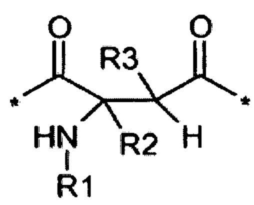

(IIb),

où

R1 représente de l'hydrogène ou un groupe fonctionnel aliphatique, cycloaliphatique ou aromatique, lequel peut également contenir des hétéroatomes, en particulier des atomes d'azote ou des atomes d'oxygène, ainsi que des groupes hydroxyle,
R2 et R3 représentent, indépendamment l'un de l'autre, de l'hydrogène ou un groupe fonctionnel aliphatique ou aromatique, et R2 et R3 peuvent également faire partie d'un système cyclique cycloaliphatique,
R4, R5, R6 et R7 représentent, indépendamment les uns des autres, de l'hydrogène ou un groupe fonctionnel aliphatique ou aromatique, et R5 et R6 peuvent également faire partie d'un système cyclique cycloaliphatique,
I représent le nombre d'atomes d'hydrogène actifs de Zerewitinoff du composé de départ H-fonctionnel,
m, n et o sont, indépendamment les uns des autres, des nombres entiers, où n, o = 0 ou ≥ 1 et m ≥ 1, et le rapport de o à 1 est en moyenne au moins 0,6

et où le poids moléculaire équivalent de la structure présentée dans la formule I n'excède pas la valeur de 18900 g/mol.

**10.** Système de polyurée-polyuréthane, comprenant
comme composant A) des prépolymères à fonctionnalité isocyanate pouvant être obtenus par mise en réaction de polyisocyanates aliphatiques et/ou aromatiques A1) avec des polyols A2),
comme composant B) un hydroxyaminopolymère selon la revendication 9,
éventuellement, comme composant C) des matières de charge organiques,
éventuellement, comme composant D) des produits de réaction de prépolymères à fonctionnalité isocyanate selon le composant A) avec des composés hydroxyaminofonctionnels selon le composant B) et/ou des matières de charge organiques selon le composant C) et
éventuellement, comme composant E) de l'eau et/ou une amine tertiaire.

**11.** Système de polyurée-polyuréthane selon la revendication 10, **caractérisé en ce qu'**un polyol trifonctionnel est utilisé pour fabriquer le composant A.

**12.** Système de polyurée-polyuréthane selon la revendication 10 ou 11, **caractérisé en ce que** le composant E) contient une amine tertiaire de formule générale (IX)

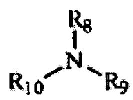

(IX)

où $R_8$, $R_9$ et $R_{10}$ peuvent être, indépendamment les uns des autres, des groupes fonctionnels alkyle ou hétéroalkyle présentant des hétéroatomes dans la chaîne alkyle ou sur leur extrémité, ou $R_8$ et $R_9$ peuvent former un hétérocycle aliphatique, insaturé ou aromatique conjointement avec l'atome d'azote les portant, lequel peut éventuellement contenir d'autres hétéroatomes.

**13.** Système de polyurée-polyuréthane selon l'une des revendications 10 à 12, **caractérisé en ce que** l'amine tertiaire est sélectionnée dans le groupe triéthanolamine, tétrakis (2-hydroxyéthyl)éthylènediamine, N,N-diméthyl-2-(4-méthylpipérazin-1-yl)éthanamine, 2-{[2-(diméthylamino)éthyl](méthyl)amino}éthanol, 3,3',3"-(1,3,5-triazinan-1,3,5-triyl)tris(N,N-diméthyl-propan-1-amine).

**14.** Système de polyurée-polyuréthane selon l'une des revendications 10 à 13 pour fermer, lier, agglutiner ou recouvrir des tissus cellulaires, ou sceller des fuites dans les tissus cellulaires.

**15.** Système de dosage à deux chambres pour un système de polyuréepolyuréthane selon l'une des revendications 10 à 13, **caractérisé en ce que** le composant A) est contenu dans ladite première chambre et le composant B) est contenu dans ladite seconde chambre, ainsi que, le cas échéant, les composants C), D) et E) du système de polyuréepolyuréthane.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009106245 A2
- WO 2010066356 A
- US 5739192 A1
- US 5597390 A1
- US 20050171002 A1
- DE 19616984 A1
- DE 19508308 A1
- WO 2010090345 A1
- JP 2009022753 A
- JP 04089860 A
- EP 1525244 A1
- US 3404109 A1
- US 3829505 A1
- US 3941849 A1
- US 5158922 A1
- US 5470813 A1
- EP 700949 A1
- EP 743093 A1
- EP 761708 A1
- WO 9740086 A1
- WO 9816310 A1
- WO 0047649 A1
- EP 10163170 A
- US 5158922 A
- JP 4145123 A
- WO 0139883 A1
- WO 0180994 A1
- US 4987271 A1
- DE 3132258 A1
- EP 406440 A1
- US 5391722 A1
- US 5099075 A1
- US 4721818 A1
- US 4877906 A1
- EP 385619 A1


### In der Beschreibung aufgeführte Nicht-Patentliteratur


- B. Handbuch Apparate. Vulkan-Verlag Essen, 1990, 188-208